# EUROPÄISCHE PATENTSCHRIFT

(11) **EP 2 403 842 B1**
(45) Veröffentlichungstag und Bekanntmachung des Hinweises auf die Patenterteilung: **29.06.2016**
(21) Anmeldenummer: 10714559.1
(22) Anmeldetag: 02.03.2010
(51) Int. Cl.: C07D 285/01

(54) **CHIRALE DISULFONIMIDE**
CHIRAL DISULFONIMIDES
DISULFONIMIDES CHIRAUX

(30) Priorität: 02.03.2009 DE 102009011055
(43) Veröffentlichungstag der Anmeldung: 11.01.2012
(73) Patentinhaber: Studiengesellschaft Kohle MbH, 45470 Mülheim an der Ruhr (DE)
(72) Erfinder: LIST, Benjamin, 45470 Mülheim an der Ruhr (DE); LAY, Frank, 45468 Mülheim an der Ruhr (DE); GARCIA-GARCIA, Pilar, 37194 Salamanca (ES)
(86) Internationale Anmeldenummer: PCT/DE2010/000226
(87) Internationale Veröffentlichungsnummer: WO 2010/099786

(56) Entgegenhaltungen:
- US-A- 4 122 088
- FARRAR W V: "Reactions of some arenesulphonyl chlorides" JOURNAL OF THE CHEMICAL SOCIETY, CHEMICAL SOCIETY, LETCHWORTH; GB LNKD- DOI:10.1039/JR9600003063, Nr. 7, 1. Juli 1960 (1960-07-01), Seiten 3063-3069, XP002186710 ISSN: 0368-1769
- GARCIA-GARCIA PILAR: "A Powerful Chiral Counteranion Motif for Asymmetric Catalysis" ANGEWANDTE CHEMIE, Bd. 48, 13. Mai 2009 (2009-05-13), Seiten 4363-4366, XP002590493
- TRESKOW, M. ET AL.: "BINBAM - A new Motif for Strong and Chiral Bronsted Acids" EUROPEAN JOURNAL OF ORGANIC CHEMISTRY, 17. Juni 2009 (2009-06-17), Seiten 3693-3697, XP002590494

## Beschreibung

Die vorliegende Erfindung betrifft chirale Disulfonimide sowie deren Salze und Metallkomplexe und deren Anwendung als Katalysatoren.

Viele chemische Transformationen werden durch Brønsted-Säuren katalysiert. Gerade in der enantioselektiven Organokatalyse ist diese Möglichkeit der metallfreien, und bei chiralen Brønsted-Säuren auch enantioselektiven Katalyse, ein schnell wachsender Zweig mit zunehmenden Anwendungen. Man unterscheidet in diesem Bereich der Organokatalyse zwischen Wasserstoffbrückenkatalysatoren, wie Thioharnstoffen sowie TADDOL- und BINOL-Derivaten, und stärkeren Brønsted-Säuren wie Phosphorsäurediester und deren Derivate. Die Möglichkeit der Aktivierung durch diese chiralen, organischen Brønsted-Säuren beschränkt sich jedoch auf reaktive Elektrophile, wie z.B. Imine und Nitroolefine, sowie vereinzelt auf Ketone und aktivierte Aldehyde. Die Herstellung sehr starker, chiraler Brønsted-Säuren zur Aktivierung von einfachen Ketonen, Aldehyden und Alkenen sind daher erstrebenswert.

In J. Am. Chem. Soc. 2006, 128, 9626-9627 führen Yamamoto et. al chirale, substituierte N-Triflylphosphoramide als starke Brønsted-Säuren zur Aktivierung von Ketonen ein. Die Darstellung einer ebenfalls starken, chiralen Brønsted-Säure, der 1,1'-Binaphthalen-2,2'-disulfonsäure, wird bereits durch Barber und Smiles in J. Chem. Soc. 1928, 1141-1149 beschrieben. Armarego und Turner untersuchen in J. Chem. Soc. 1957, 13-22 die optische Aktivität und optische Stabilität dieser Säure. Die japanischen Patente 2005-132815, 2005-132816 und 2005-134365 offenbaren die enantiomerenreine Darstellung dieser Säure, den Einsatz als Reagenz zur Racematspaltung und die Anwendung als NMR-Shift-Reagenz. List et al. verwendeten diese Säure als chiralen Brønsted-Säurekatalysator in Adv. Synth. Catal. 2008, 350, 962-966 und Chem. Asian. J. 2008, 3, 430-437, jedoch ohne Enantioselektivität zu erlangen. Ishihara et al. benutzten die 1,1'-Binaphthalen-2,2'-disulfonsäure in Kombination mit substituierten Pyridinen in J. Am. Chem. Soc. 2008, 130, 16858-16860 als chirale Brønsted-Säure-Basen Organokatalysatoren.

Gleichermaßen ist das unbekannte cyclische Disulfonimid der 1,1'-Binaphthalen-2,2'-disulfonsäure, dessen substituierte Derivate und die substituierten Derivate der 1,1'-Binaphthalen-2,2'-disulfonsäure als starke Brønsted-Säure einzustufen. Die konjugierten Basen der Disulfonsäuren und Disulfonimide eignen sich ebenfalls als chirale Anionen in enantioselektiven Gegenanion vermittelter Katalyse und in der Lewis-Säure Katalyse.

Der vorliegenden Erfindung lag demgemäß die Aufgabe zugrunde chirale Disulfonimide und ein einfaches Verfahren zur Herstellung chiraler Disulfonimide zur Verfügung zu stellen sowie deren Anwendung in der Katalyse.

Gegenstand der vorliegenden Erfindung sind demgemäß chirale Disulfonimide mit der allgemeinen Formel IV 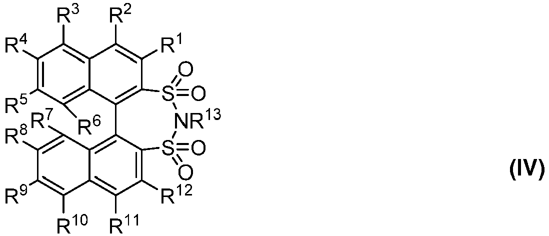 in der
R¹, R², R³, R⁴, R⁵, R⁶, R⁷, R⁸, R⁹, R¹⁰, R¹¹ und R¹² gleich oder verschieden sein können und unabhängig voneinander H, OH, F, Cl, Br, I, CN, NO₂, NO, SO₂, SO₃H, NH₂, PH₃, COOH, SO₃X, COOY, worin X und Y für Na oder K steht, eine C₁-C₂₀-Alkyl-, C₂-C₂₀-Alkenyl- oder C₂-C₂₀-Alkinyl-, Aryl-, Aryl-(C₁-C₆)-alkyl, Hetero(C₁-Cy₆)-alkyl-, Heteroaryl-Gruppe, die ggf. substituiert sein können, bedeuten,
R¹³ H, C₁-C₆-Alkyl-, C₂-C₆-Alkenyl- oder C₂-C₆-Alkinyl-, Gruppe Aryl-, Aryl-(C₁-C₆)-alkyl, Hetero(C₁-C₆)-alkyl-, Heteroaryl oder SiR¹⁴R¹⁵R¹⁶ steht, wobei R¹⁴, R¹⁵, R¹⁶ für C₁-C₆-Alkyl-, C₂-C₆-Alkenyl- oder C₂-C₆-Alkinyl-, Aryl-, Aryl-(C₁-C₆)-alkyl, Hetero-(C₁-C₆)-alkyl-, Heteroaryl, die ggf. substituiert sein können, steht,
sowie deren organische Salze, Metallsalze und Metallkomplexe.

In der organischen Synthese, insbesondere in der Synthese von pharmazeutischen Wirkstoffen werden chirale Verbindungen häufig als Katalysatoren eingesetzt, um das gewünschte Produkt in einer hohen Enantiomerenreinheit bzw. Diastereomerenreinheit zu erhalten. Für den Einsatz als Katalysatoren in der organischen Synthese haben sich Verbindungen als besonders geeignet erwiesen, in denen sich die Reste in den Verbindungen von BINOL ableiten. Diese erfindungsgemäßen Verbindungen sind in der allgemeinen Formel IV dargestellt 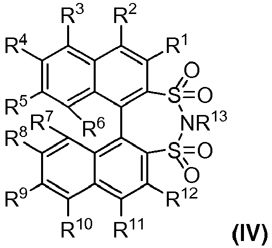 in der
R¹, R², R³, R⁴, R⁵, R⁶, R⁷, R⁸, R⁹, R¹⁰, R¹¹ und R¹² gleich oder verschieden sein können und unabhängig voneinander H, OH, F, Cl, Br, I, CN, NO₂, NO, SO₂, SO₃H, NH₂, PH₃, COOH, SO₃X, COOY, worin X und Y für Na oder K steht, eine C₁-C₂₀-Alkyl-, C₂-C₂₀-Alkenyl- oder C₂-C₂₀-Alkinyl-, Aryl-, Aryl-(C₁-C₆)-alkyl, Hetero(C₁-Cy₆)-alkyl-, Heteroaryl-Gruppe, die ggf. substituiert sein können, bedeuten,
R¹³ für H, C₁-C₆-Alkyl-, C₂-C₆-Alkenyl- oder C₂-C₆-Alkinyl-, Gruppe Aryl-, Aryl-(C₁-C₆)-alkyl, Hetero(C₁-C₆)-alkyl-, Heteroaryl, oder SiR¹⁴R¹⁵R¹⁶, Br, Cl, I, F steht, wobei R¹⁴, R¹⁵, R¹⁶ für C₁-C₆-Alkyl-, C₂-C₆-Alkenyl- oder C₂-C₆-Alkinyl-, Aryl-, Aryl-(C₁-C₆)-alkyl, Hetero-(C₁-C₆)-alkyl-, Heteroaryl, die ggf. substituiert sein können, steht,
sowie deren organische Salze, Metallsalze und Metallkomplexe.

Gegenstand der Erfindung sind auch Verbindungen mit den folgenden allgemeinen Formeln VI und VII: 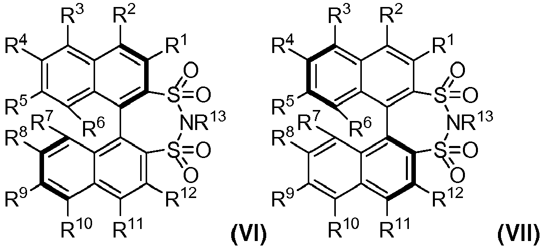 in denen die Reste R¹, R², R³, R⁴, R⁵, R⁶, R⁷, R⁸, R⁹, R¹⁰, R¹¹ und R¹², sowie R¹³ wie oben definiert sind, wobei die Enantiomeren in einem Verhältnis von 98 : 2 bis 100:0, d.h. in enatiomerenreiner Form, vorliegen.

Es wurde festgestellt, dass die erfindungsgemäßen Verbindungen sich gut als Katalysatoren für die enantioselektive Synthese eignen. Hierbei fungieren sie als chirale Brønsted-Säuren bzw. mit R¹³ = SiR¹⁴R¹⁵R¹⁶ als Lewis-Säuren oder deren konjugierte Base als chirale Anionen in enantioselektiven Gegenanion vermittelten Katalysen.

Die nachfolgenden Definitionen für die einzelnen Reste gelten für die Reste R¹, R², R³, R³, R⁴, R⁵, R⁶, R⁷, R⁸, R⁹, R¹⁰, R¹⁰, R¹¹, R^{12'} und den Rest R¹³.

C₁-C₂ₒ-Alkyl kann unverzweigt (linear) oder verzweigt sein und hat 1, 2, 3, 4, 5, 6, 7, 8, 9, 10, 11, 12, 13, 14, 15, 16, 17, 18, 19 oder 20 Kohlenstoffatome. Alkyl ist vorzugsweise C₁-C₆-Alkyl, insbesondere Methyl, Ethyl, Propyl, Isopropyl, Butyl, Isobutyl, sec-Butyl oder tert.-Butyl, ebenso Pentyl, 1-, 2- oder 3-Methylpropyl, 1,1-, 1,2- oder 2,2 Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl. Bevorzugte substituierte Alkylreste sind Trifluormethyl, Pentafluroethyl und 1,1,1-Trifluorethyl.

Cycloalkyl ist vorzugsweise Cyclopropyl, Cyclobutyl, Cyclopentyl, Cyclohexyl oder Cycloheptyl. Alkylen bedeutet vorzugsweise Methylen, Ethylen, Propylen, Butylen, Pentylen oder Hexylen, aber auch verzweigtes Alkylen.

Alkylen ist bevorzugt Vinyl.

Alkinyl ist bevorzugt C=CH.

Halogen ist F, Cl, Br oder I.

Alkoxy ist vorzugsweise Methoxy, Ethoxy, Propoxy oder Butoxy.

C₃-C₈-Heterocycloalkyl mit einem oder mehreren Heteroatomen ausgewählt aus N, O und S ist vorzugsweise 2,3-Dihydro-2-, -3-, -4- oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1-,-2-, -3-, -4- oder -5-pyrrolyl, 2,5-Dihydro-1-, -2-, -3-, -4- oder -5-pyrrolyl, 1-, 2- oder 3-pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-piperidinyl, 2-, 3- oder 4-morpholinyl, Tetrahydro-2-, -3- oder -4-pyranyl, 1,4-Dioxanyl, 1,3-Dioxan-2-, -4- oder -5-yl, hexahydro-1-, -3- oder -4-pyridazinyl, Hexahydro-1-, -2-, -4- oder -5-pyrimidinyl, 1-, 2- oder 3-piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8-3,4-Dihydro-2H-benzo-1,4-oxazinyl.

Gegebenenfalls substituiert bedeutet unsubstituiert oder mono-, di-, tri-, tetra- oder pentasubstituiert.

Aryl ist vorzugsweise Phenyl, Naphthyl oder Diphenyl.

Arylalkyl ist vorzugsweise Benzyl.

Heteroaryl mit einem oder mehreren Heteroatomen ausgewählt aus N, O und S ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4-oder 5-Thiazolyl, 3-, 4- oder 5-Isothiazolyl, 2-, 3- oder 4-Pyridyl, 2-, 4-, 5- oder 6-Pyrimidinyl, außerdem bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2-oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo-1,4-oxazinyl, außerdem bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

In einer bevorzugten Ausführungsform der vorliegenden Erfindung ist mindestens einer von R¹ oder R¹² bzw. R^{1'} oder R^{12'} nicht Wasserstoff und ist ausgewählt aus Phenyl-, 2,4,6-Triisopropyl-phenyl, Mesityl, 9-Phenanthryl, 9-Anthracenyl, Ferrocenyl, N-(perfluorophenyl)acetamid, N-(4-chlorophenyl)acetamid, N-(naphthalen-1-yl)acetamid, N-benzhydrylacetamid, N-(2,6-diisopropylphenyl)acetamid, 1-Anthracenyl, Corannulen, Porphyrin, 1-Naphthyl, 2-Naphthyl, 4-Biphenyl, 3,5-(Trifluoromethyl)-phenyl, 2,6-Dimethylphenyl, tert-Butyl, tris-Mesityl-silyl, tris-Phenyl-silyl, 4-Nitrophenyl und 2,6-Methyl-4-butylphenyl, Trifluormethyl, unverzweigte (lineare) und verzweigte (C₁-C₁₂-Perfluoralkyle, 3,4,5-Trifluorphenyl, 1,3-bis(perfluoropropan-2-yl)-phenyl, 1,3-bis(perfluorobutyl)-phenyl und/oder Pentafluorphenyl sowie Chlorid, Jodid, Fluorid, B(OH)₂, B(alkyl)₂, B(Oalkyl)₂, B(pinakol), BF₃X X=Na oder K, OTf, MgCl, MgBr, ZnCl. Die anderen Reste sind vorzugsweise Wasserstoff.

In einer weiteren bevorzugten Ausführungsform der vorliegenden Erfindung ist mindestens einer der Reste R⁴ und R⁹ bzw. R^{4'}, R^{4"}, R^{9'} und R^{9"} ausgewählt aus NO₂ oder I.

Die erfindungsgemäßen Verbindungen können in an sich bekannter Weise dem Fachmann gut bekannten Verfahrensschritten in organische Salze, Metallsalze bzw. Metallkomplexe überführt werden. In einer möglichen Ausführungsform werden die Disulfonimide mit einem entsprechenden Metallsalz umgesetzt, beispielsweise mit dem Carbonat des entsprechenden Metalles.

Beispiele für organische Salze, Metallsalze bzw. Metallkomplexe sind im nachfolgenden Schema dargestellt:

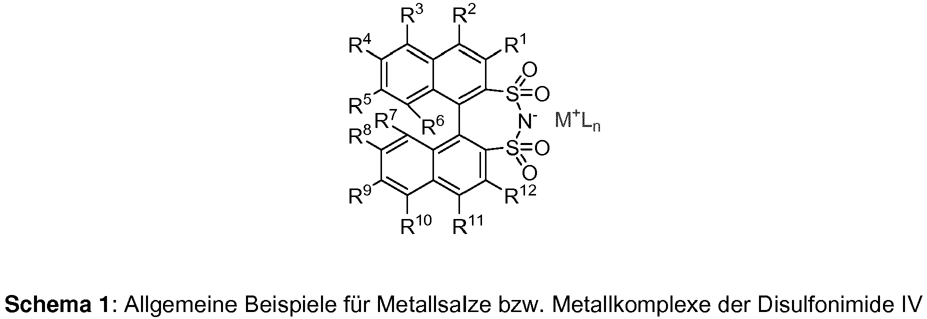

Im Schema 1 können beliebige Metalle oder organische Kationen wie z.B. tertiäre Ammoniumionen für M stehen. L bezeichnet beliebige Liganden in der Anzahl n = 0-10. Auch wenn die Verbindungen im Schema 1 als Salze dargestellt sind, ist die genaue Struktur mit Metallen nicht bekannt, sie können auch die Struktur von Metallkomplexen aufweisen. Es wird daher im Rahmen der vorliegenden Erfindung die Formulierung Metallsalze oder Metallkomplexe verwendet. Die Metallverbindungen sind nicht auf bestimmte Metallverbindungen oder -komplexe beschränkt. Geeignete Metallverbindungen leiten sich von Li, Na, K, Rb, Cs, Be, Mg, Ca, Sr, Ba Sc, Ti, V, Cr, Mn, Fe, Co, Ni, Cu, Zn, Y, Zr, Mo, Ru, Rh, Pd, Ag, W, Re, Os, Ir, Pt, Au, AI, Pb, La, Sm, Eu, Yb ab.

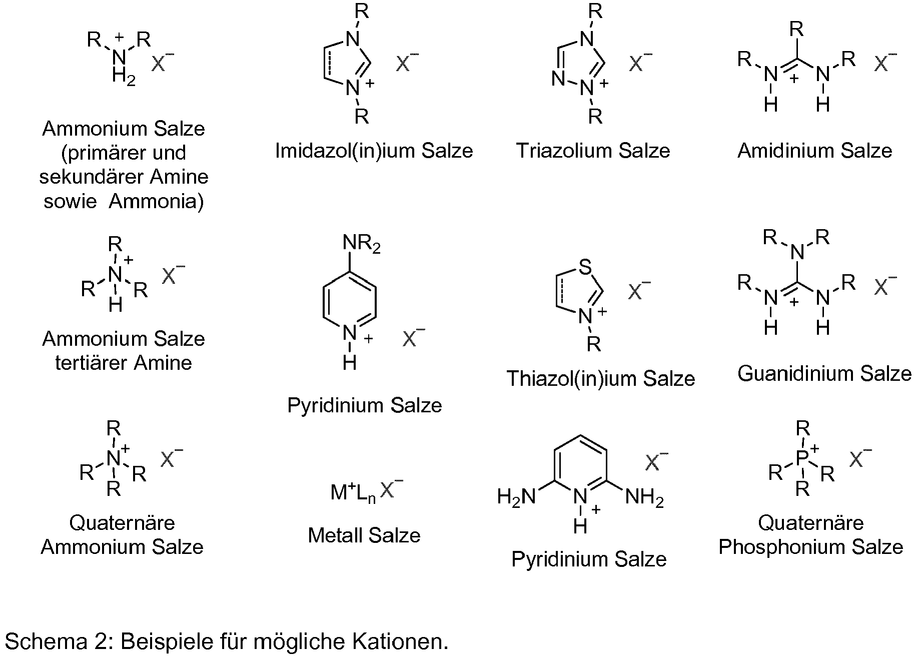

Ein bevorzugtes Herstellungsverfahren der vorliegenden Erfindung ist ein Verfahren zur Herstellung von substituierten Disulfonimiden mit mit chiralem BINOL Rückgrat: 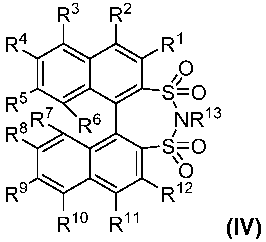 in der
R¹, R², R³, R⁴, R⁵, R⁶, R⁷, R⁸, R⁹, R¹⁰, R¹¹, R¹² und R¹³ wie oben definiert sind, worin
   (A) ein Binolderivat mit der allgemeinen Formel X mit der allgemeinen Formel XI 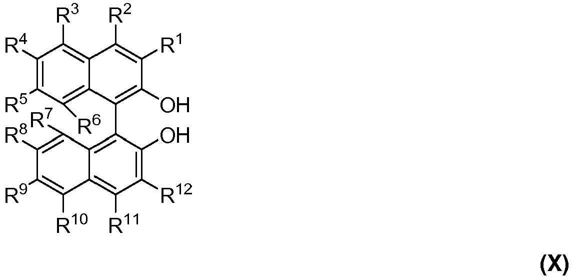 mit einem Thiocarbamoylchlorid der allgemeinen Formel XII 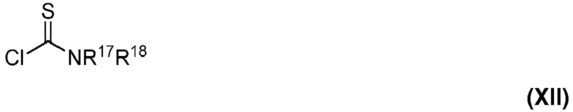 in der R¹⁷ und R¹⁸ gleich oder verschieden sein können und für eine C₁-C₆-Alkylgruppe stehen,
      unter Bildung eines *O*-Arylthiocarbamates mit der allgemeinen Formel XIII. 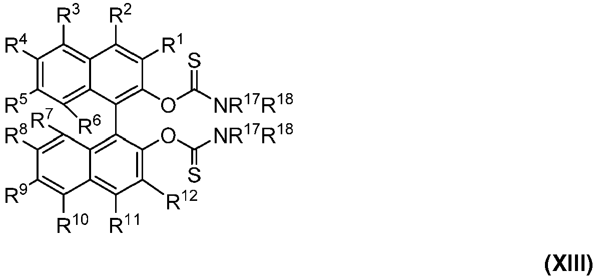 umgesetzt wird,
   (B) das *O*-Arylthiocarbamat mit der Formel XII in das entsprechende S-Arylthiocarbamat mit der Formel 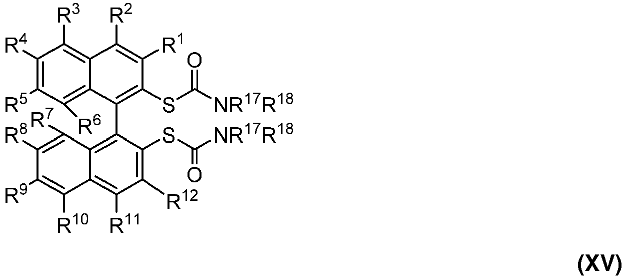 umgewandelt wird, und
   (C) das *S*-Arylthiocarbamat in Gegenwart eines Oxidationsmittels in die Verbindungen mit der Formel XVII umgewandelt wird. 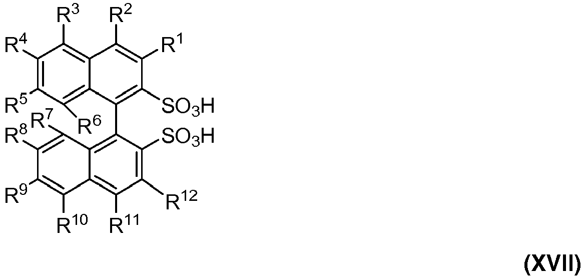 In einem Verfahrensschritt (D) werden die Sulfonsäuren mit der Formel XVII in an sich bekannter Weise in die Säurehalogenide mit der Formel XIX wobei R¹⁹ = Cl, Br, I oder F. 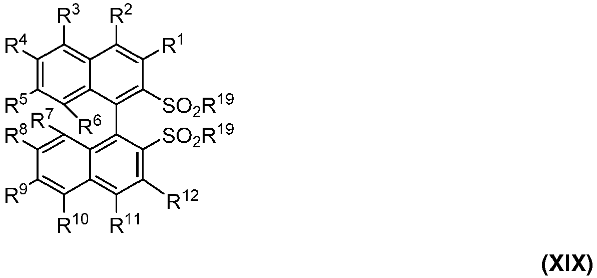 überführt und in einem nachfolgenden Verfahrensschritt
   (E) anschließend mit Ammoniak oder einem primären Amin in das entsprechende Imid mit der Formel IV bzw. V umgewandelt werden.

Alternativ kann in dieser Syntheseroute das S-Arylthiocarbamat mit der Formel XV bzw. XVI in einem dem Fachmann bekannten Verfahrensschritt (D1) direkt in die Säurehalogenide mit der Formel XIX umgewandelt werden, wobei R¹⁹ = Cl, Br, I oder F.

In einer alternativen Syntheseroute geht man von einem 3,3'-unsubstituiertem Disulfonimid mit der allgemeinen Formel IVa aus (R¹, R¹² = H bzw. R^{1'}, R^{12'} = H), welches nach obiger Syntheseroute dargestellt werden kann.

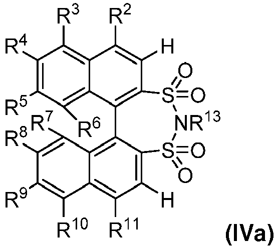

Diese wird in einem dem Fachmann bekannten Verfahrenschritt F) in ein 3,3'-Dihalogenid (R¹, R¹² = Cl, Br, I oder F bzw. R^{1'}, R^{12'} = Cl, Br, I oder F) oder ein 3,3'-Ditriflat (R¹, R¹² = OTf bzw. R^{1'}, R^{12'} = OTf) mit der allgemeinen Formel XX umgewandelt.

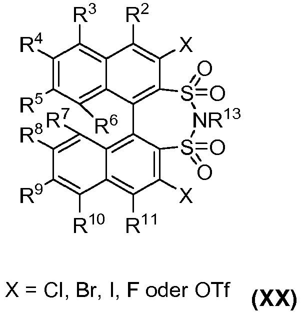

Alternativ können in dieser Syntheseroute die Disulfonimide IVain einem dem Fachmann bekannten Verfahrensschritt G) in ein 3,3'-Diboronat (R¹, R¹² = B(OH)₂, B(alkyl)₂, B(Oalkyl)₂, B(pinakol), BF₃X X=Na, K bzw. R^{1'}, R^{12'} = B(OH)₂, B(alkyl)₂, B(Oalkyl)₂, B(pinakol), BF₃X X=Na, K) mit der allgemeinen Formel XXII umgewandelt werden.

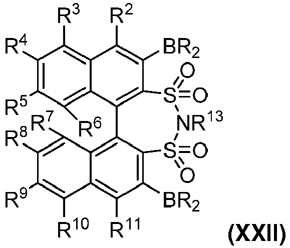

Die Halogenide und Ditriflate mit der allgemeinen Formel XX bzw. XXI oder die Boronate mit der allgemeinen Formel XXII bzw. XXIII dienen als Ausgangsubstanzen für dem Fachmann wohlbekannte Kupplungsreaktionen als Verfahrensschritt H) zu den Imiden mit der Formel IV bzw. V.

Die einzelnen Reaktionsschritte des erfindungsgemäßen Verfahrens stellen aus dem Stand der Technik bekannte Verfahren dar. Im Verfahrensschritt A wird die Dihydroxyverbindung mit der Formel IVmit einem Thiocarbamoylchlorid der Formel XII in Gegenwart eines Hydrids in einem Lösungsmittel bei erhöhter Temperatur umgesetzt. Als Lösungsmittel kommt jedes organische Lösungsmittel in Betracht, dass die Reaktion nicht nachteilig beeinflusst.

Der Reaktionsschritt B erfolgt vorzugsweise in einem geeigneten Lösungsmittel oder lösungsmittelfrei durch Erhitzen des in Schritt A erhaltenen *O*-Arylthiocarbamates. Das erhaltene S-Arylthiocarbamat mit der Formel XV wird im nächsten Verfahrensschritt C in eine Sulfonsäure mit der Formel XVII umgewandelt. Dieser Verfahrensschritt erfolgt vorzugsweise in Gegenwart eines Oxidationsmittels wie H₂O₂ und einer Säure und/oder einer organischen Persäure.

Die Umwandlung der Sulfonsäure mit der Formel XVII in das Säureimid mit der Formel IV kann in an sich bekannter Weise nach dem Fachmann gut bekannten Verfahrensschritten erfolgen.

Alternativ kann in dieser Syntheseroute das S-Arylthiocarbamat mit der Formel XV in einem dem Fachmann bekannten Verfahrensschritt (D1) direkt in die Säurehalogenide mit der Formel XVII umgewandelt werden, wobei R¹⁹ = Cl, Br, I oder F

Ein Beispiel für eine allgemeine Syntheseroute von BINOL X zu Disulfonimid IV ist Schema 3 zu entnehmen. Ausgangssubstanzen sind entsprechende substituierte bzw. unsubstituierte BINOLE X, diese werden mit Dimethylthiocarbamoylchlorid in das entsprechende *O*-Arylthiocarbamat XIII umgewandelt. Durch eine Newman-Kwart Umlagerung erhält man das S-Arylthiocarbamat XV, welches unter oxidativen Bedingungen zur Disulfonsäure XVII umgesetzt wird. Umwandlung dieser Säure in die entsprechenden Sulfonsäurehalogenide XIX und Ringschluß mit Ammoniak ergeben das Disulfonimid IV.

Alternativ kann in dieser Syntheseroute das S-Arylthiocarbamat mit der Formel XV in einem dem Fachmann bekannten Verfahrensschritt (D1) direkt in die Säurehalogenide mit der Formel XIX umgewandelt werden. Ringschluß mit Ammoniak ergeben das Disulfonimid IV. Eine allgemeine Beschreibung zur Darstellung der Disulfonimide ist im Experimentellen Teil gegeben.

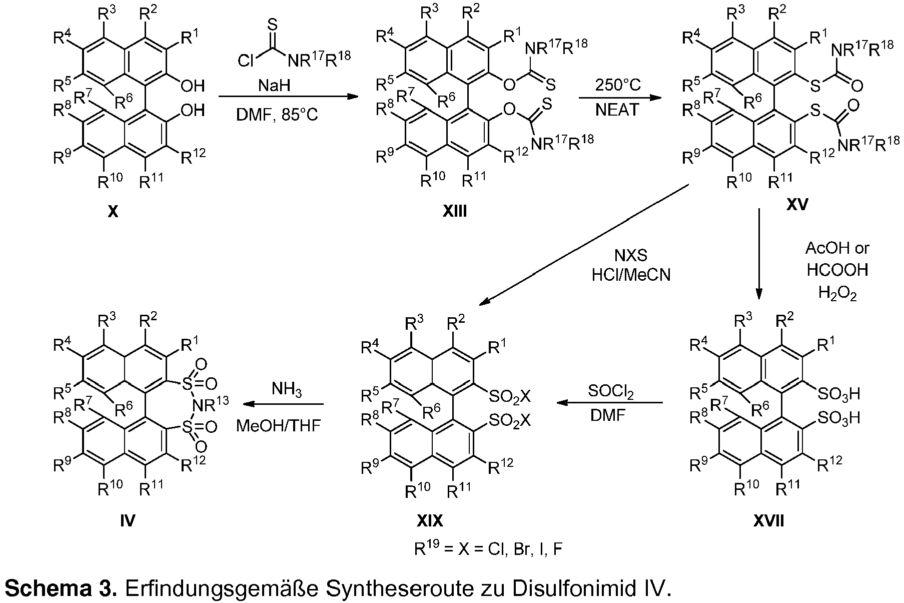

In einer alternativen Syntheseroute sind die nach obiger Route dargestellten Disulfonimide IVₐ aus (R¹, R¹² = H bzw. R^{1'}, R^{12'} = H) als Ausgangssubstanzen zu sehen. Die Disulfonimide IVa werden nach einem dem Fachmann gut bekannten Verfahrensschritt in die Halogenide oder Ditriflate mit der allgemeinen Formel XX oder die Boronate mit der allgemeinen Formel XXII umgewandelt. Diese dienen als Ausgangsubstanzen für Kupplungsreaktionen zu den Imiden mit der Formel IV. Eine Variation von R¹ und R¹² über die Disulfonimide IVa ist auch möglich, in dem man die über eine dirigierende ortho-Metallierung *in situ* gebildete Metallspezies (R¹, R¹² = Li, Mg, Zn, Cu) nicht mit Halogenquellen quencht sondern mit anderen, geeigneten Elektrophilen wie z. B. CO₂, Perfluralkyliodiden oder Isocyanaten, Aldehyden oder Ketonen.

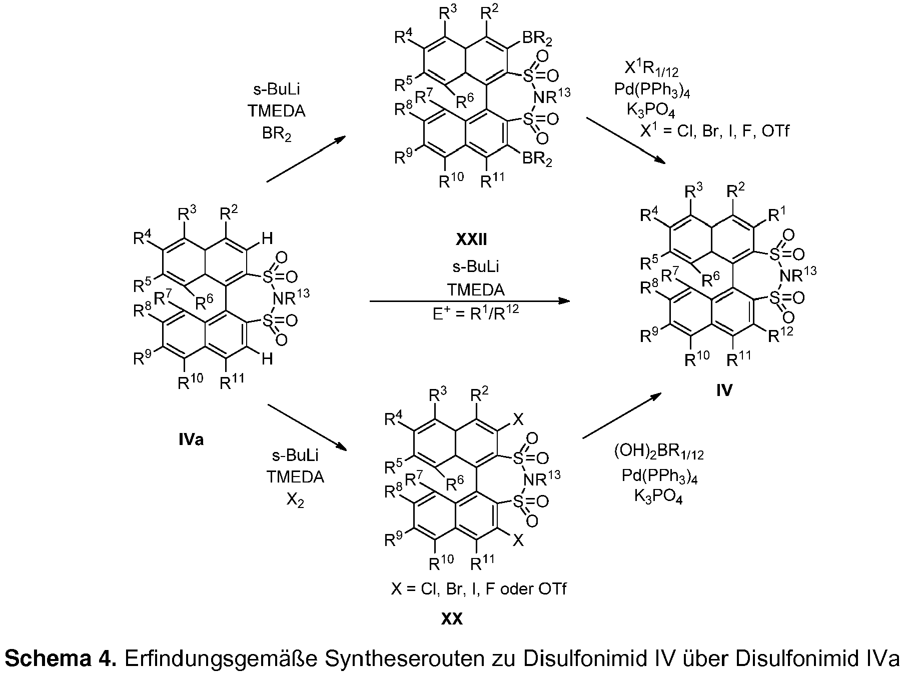

Die erfindungsgemäßen Disulfonimde sowie deren organischen Salze, Metallsalze und Metallkomplexe eignen sich besonders als starke, chirale Brønsted-Säure-Katalysatoren bzw. chirale Lewis-Säure-Katalysatoren für eine Vielzahl von Reaktionen, insbesondere zur Aktivierung von Ketonen, Aldehyden und Alkenen. Eine besonders gute katalytische Aktivität zeigen Verbindungen in denen R¹³ für H, F, Cl, Br, I oder SiR¹⁴R¹⁵R¹⁶, steht, wobei R¹⁴, R¹⁵, R¹⁶ für C₁-C₆-Alkyl-, C₂-C₆-Alkenyl- oder C₂-C₆-Alkinyl ist. Besonders bevorzugt steht R¹³ für SiR¹⁴R¹⁵R¹⁶ oder I.

Zu den Reaktionen, in denen die erfindungsgemäßen Verbindungen als Katalysatoren eingesetzt werden können, zählen Reaktionen wie Aldol-Reaktionen, vinyloge Aldol-Reaktionen, Mukaiyama-Aldol-Reaktionen, vinyloge Mukaiyama-Aldol-Reaktionen Mukaiyama-Michael-Reaktionen, Michael-Additionen, Mannich-Reaktionen, TMSCN Additionen an Aldehyde und Ketone, Veresterungen, Veretherungen, Pinakol-Umlagerungen, Acetalisierungen und verwandte Reaktionen, Cycloadditionen, Hydroaminierungen, Hydroalkoxilierung, Hydratisierungen, Olefinaktivierungen im Allgemeinen, Friedel-Crafts-Reaktionen, Epoxidöffnungen, Ritter-Reaktionen, nucleophile Substitutionen von Alkoholen, asymmetrische Ringöffnungen, asymmetrische Reduktionen, transfer Hydrierungen, Alkin-Additionen, Allylierungen, Epoxidierungen, Olefin-Metathese, Isomerisierungen, Iminiumkatalyse und Enaminkatalyse.

Eine Auswahl dieser Reaktionen ist in Schema 5 und 6 dargestellt.

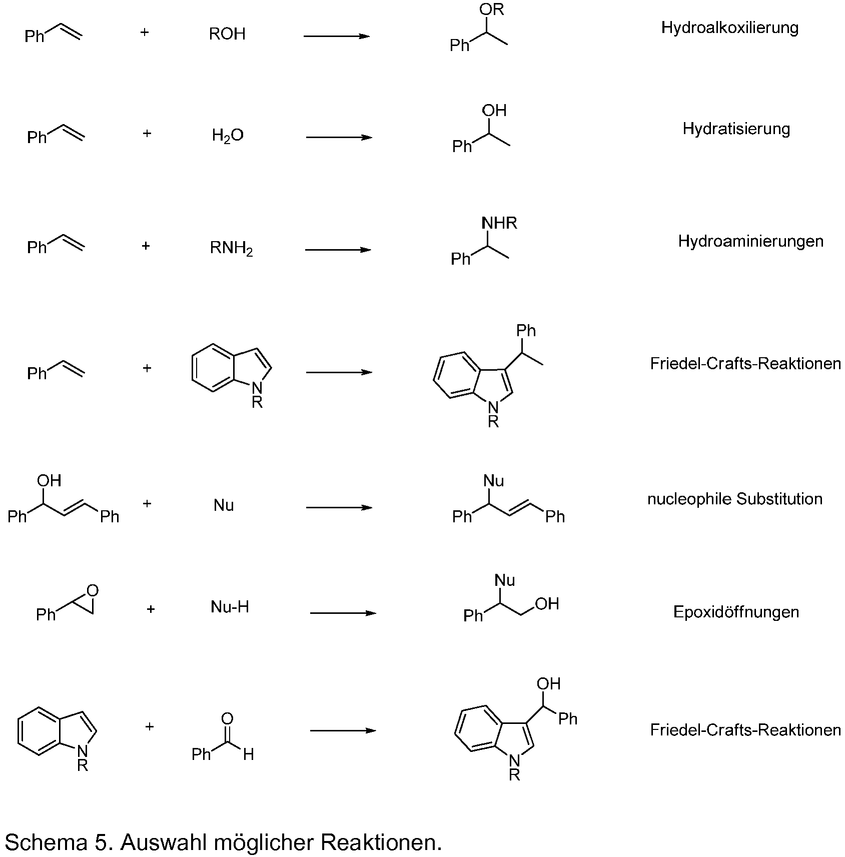

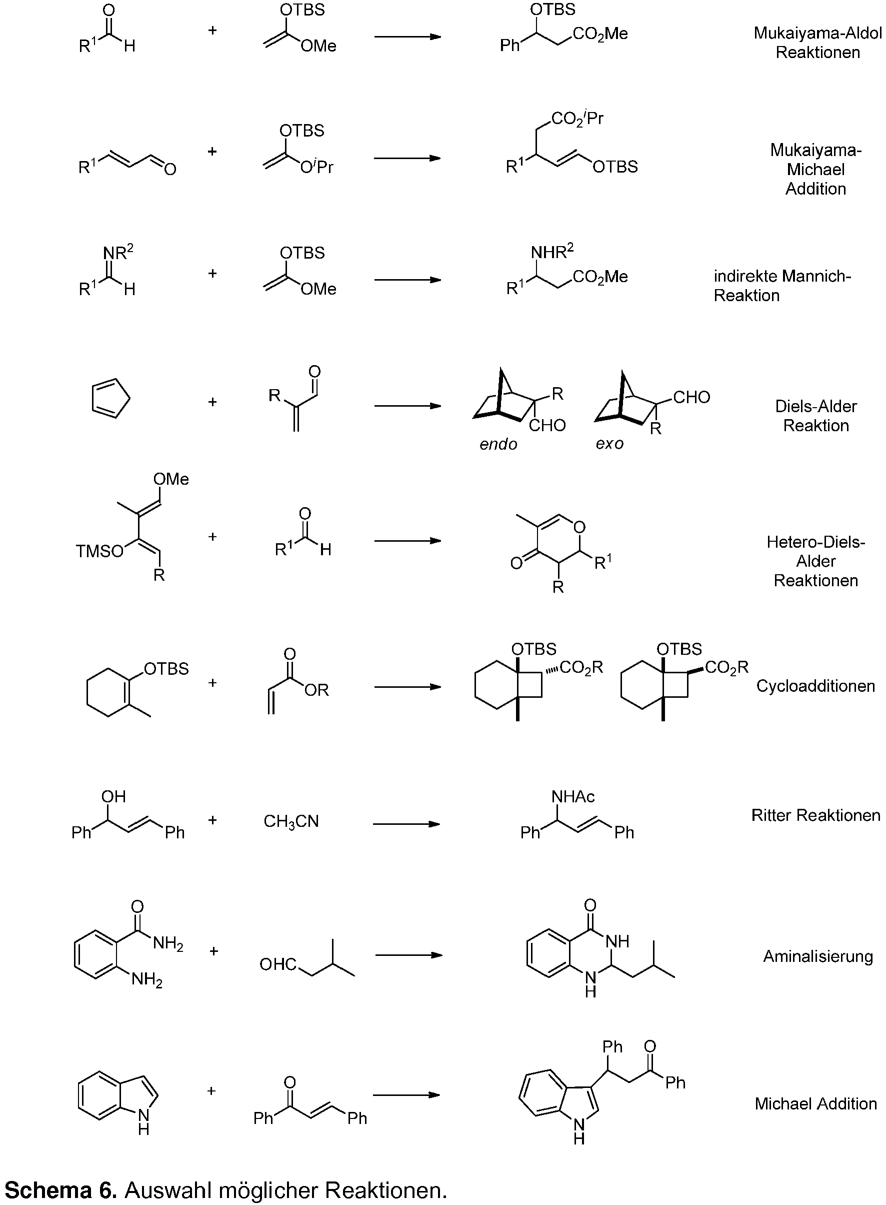

Exemplarisch sind erste Ergebnisse, die in der Mukaiyama-Aldol-Reaktion mit Disulfonimid VIIa erlangt wurden, die in Schema 9 dargestellt sind.

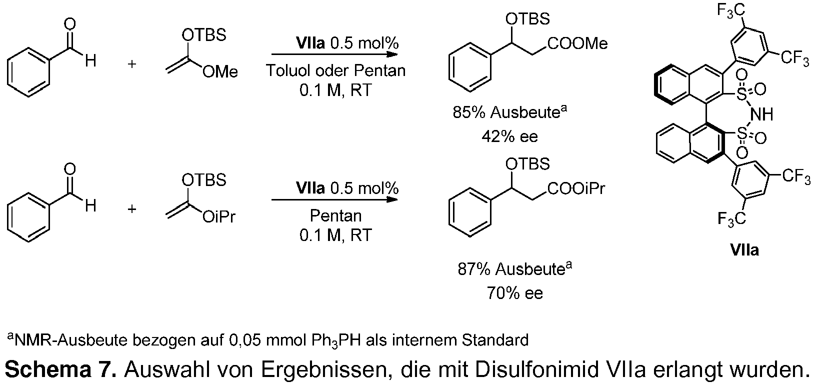

Weitere Anwendungsgebiete der erfindungsgemäßen Verbindungen sind
a) als Liganden für Metallkomplexe; exemplarisch sei der Austausch der beiden Chlorid-Liganden durch die erfindungsgemäßen Disulfonimide in den bekannten Grubbs-Katalysatoren und Hoveyda-Grubbs-Katalysatoren erwähnt.
b) in deprotonierter Form eignen sich die Disulfonimde mit den allgemeinen Formeln bis III auch als chirales Anion in der asymmetrischen Synthese.

Ein weiterer Gegenstand der vorliegenden Erfindung ist die Verwendung der erfindungsgemäßen Verbindungen als NMR-Shift-Reagenzien und als Reagenzien zur Racematspaltung.

### Experimenteller Teil

### Allgemeine Vorschrift zur Mukaiyama-Aldol-Reaktion bei Raumtemperatur

Disulfonimid VIIa (0.41 mg, 0.5 mol%) und Benzaldehyd (10µL, 0.1 mmol) werden in 0.5 mL Toluol oder Pentan bei Raumtemperatur vorgelegt. Man tropft tert-Butyl(1-methoxyvinyloxy)dimethylsilan bzw. tert-Butyl(1-isopropoxyvinyloxy)dimethylsilan (0.11 mmol) zu und rührt für 1-2 h bei Raumtemperatur. Danach wird das Lösungsmittel unter vermindertem Druck entfernt und 0.05 mmol Ph₃PH als interner Standard für eine NMR-Messung zur Ausbeutebestimmung hinzugegeben. Man löst in ca. 1 mL CDCl₃, und entnimmt 0,5-0,75 mL für eine NMR-Messung. Die restliche Lösung wird zur präparativen Dünnschichtchromatographie verwendet. Mit dem isolierten Produkt aus dieser Dünnschichtchromatographie wird der Enantiomerenüberschuß mittels chiraler GC- oder chiraler HPLC-Messung bestimmt.

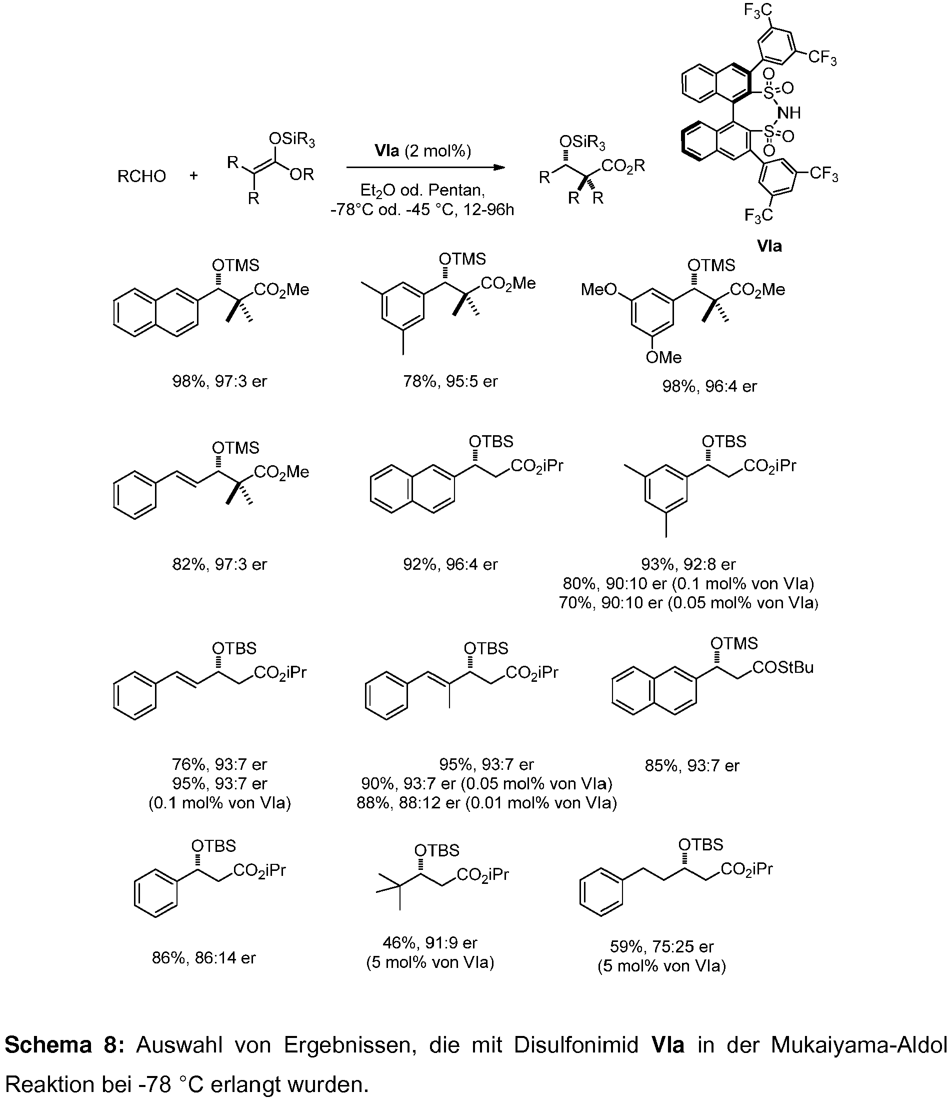

### Allgemeine Synthesevorschrift zur vinylogen Mukaiyama-Aldol-Reaktion

Disulfonimid **VIa** (2.05 mg, 5 mol%) und der entsprechende Aldehyd (0.05 mmol, 1 eq) werden in 0.25 ml Et₂O bei -78 °C vorgelegt. Man tropft (Z)-tert-butyl((1-ethoxybuta-1,3-dien-1-yl)oxy)dimethylsilane (0.065 mmol, 1.3 eq) zu und rührt für 72 h bei -78 °C. Danach quencht man mit ges. NaHCO₃- Lösung (0.25 mL) und verdünnt mit Dichlormethan (5 mL). Die Lösung wird über Na₂SO₄ getrocknet. Danach wird das Lösungsmittel unter vermindertem Druck entfernt und 0.05 mmol Ph₃PH als interner Standard für eine NMR-Messung zur Ausbeutebestimmung hinzugegeben. Man löst in ca. 1 mL CDCl₃, und entnimmt 0,5-0,75 mL für eine NMR-Messung. Die restliche Lösung wird zur präparativen Dünnschichtchromatographie verwendet. Mit dem isolierten Produkt aus dieser Dünnschichtchromatographie wird der Enantiomerenüberschuß mittels chiraler GC- oder chiraler HPLC-Messung bestimmt.

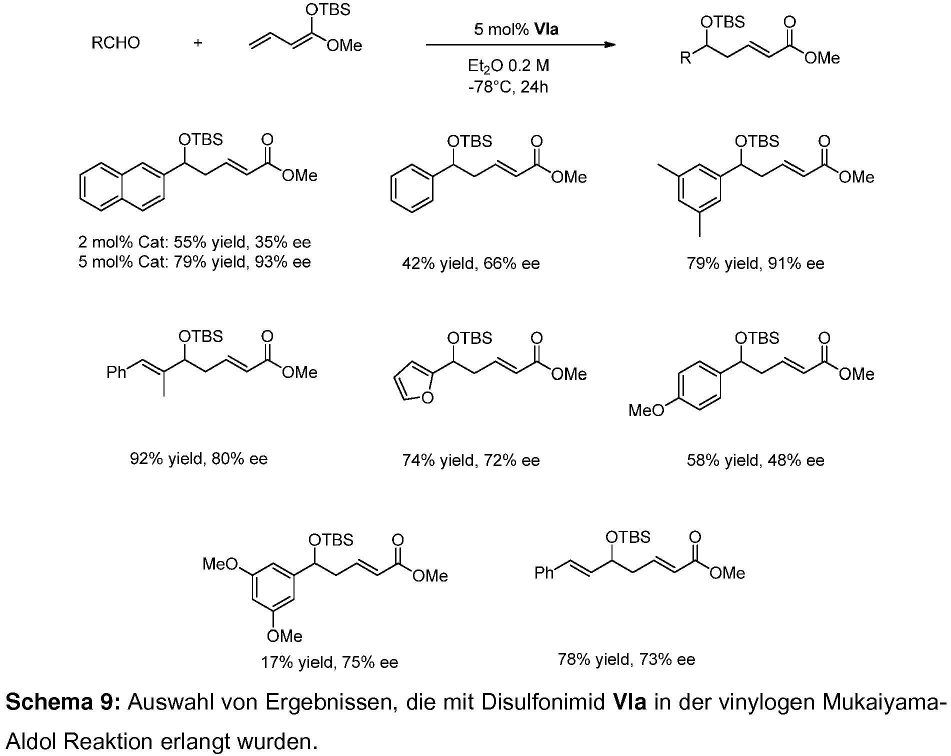

### Allgemeine Synthesevorschrift zur Allylierung von Aldehydenr

Disulfonimid VIa (2.05 mg, 5 mol%) und der entsprechende Aldehyd (0.05 mmol, 1 eq) werden in 0.25 ml Et₂O bei -78 °C vorgelegt. Man tropft Trimethyl(2-methylallyl)silan (0.065 mmol, 1.3 eq) zu und rührt für 12-16 h bei -78 °C. Danach quencht man mit ges. NaHCO₃-Lösung (0.25 mL) und verdünnt mit Dichlormethan (5 mL). Die Lösung wird über Na₂SO₄ getrocknet. Danach wird das Lösungsmittel unter vermindertem Druck entfernt und 0.05 mmol Ph₃PH als interner Standard für eine NMR-Messung zur Ausbeutebestimmung hinzugegeben. Man löst in ca. 1 mL CDCl₃, und entnimmt 0,5-0,75 mL für eine NMR-Messung. Die restliche Lösung wird zur präparativen Dünnschichtchromatographie verwendet. Mit dem isolierten Produkt aus dieser Dünnschichtchromatographie wird der Enantiomerenüberschuß mittels chiraler GC- oder chiraler HPLC-Messung bestimmt.

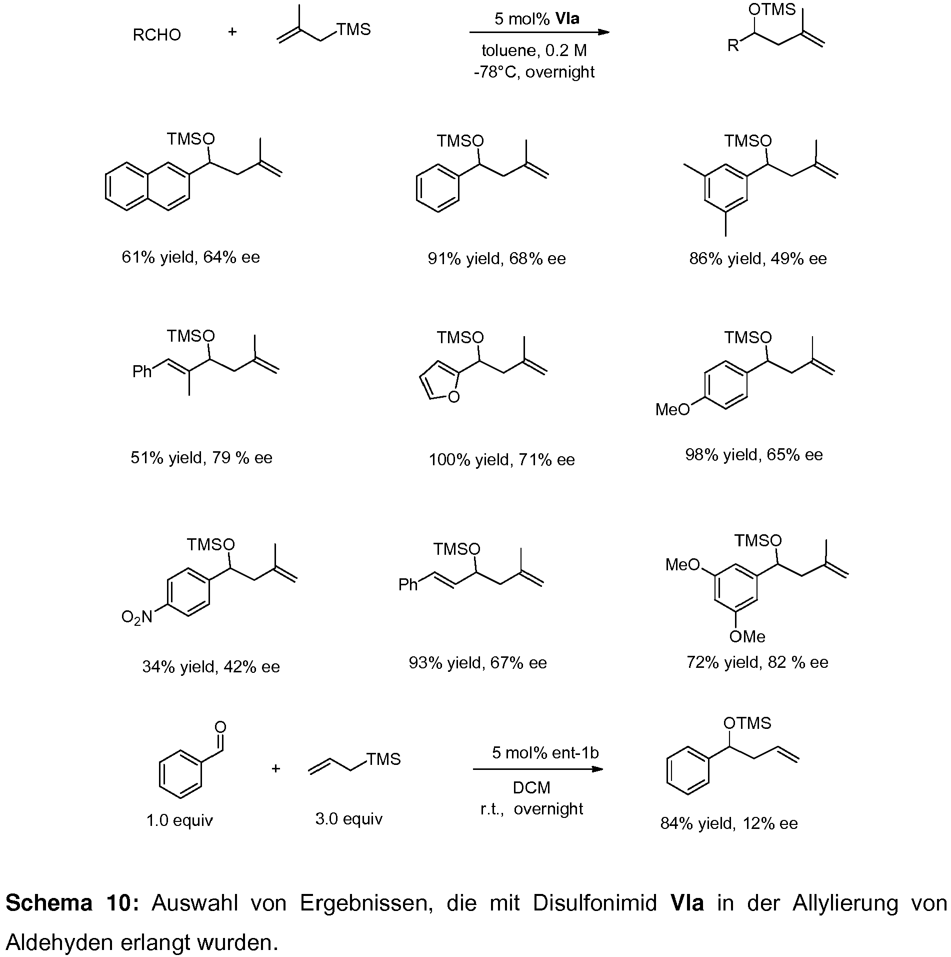

### Allgemeine Synthesevorschrift zur Hetero-Diels-AlderReaktion

Disulfonimid **VIa** (2.05 mg, 5 mol%) und der entsprechende Aldehyd (0.05 mmol, 1 eq) werden in 0.25 ml Et₂O bei -78 °C vorgelegt. Man tropft (E)-((4-methoxybuta-1,3-dien-2-yl)oxy)trimethylsilan (0.06 mmol, 1.2 eq) zu und rührt bei -78 °C. Danach quencht man mit 10%iger TFA Lösung in Dichlormethan bei -78 °C. Das Lösungsmittel wird unter vermindertem Druck entfernt und 0.05 mmol Ph₃PH als interner Standard für eine NMR-Messung zur Ausbeutebestimmung hinzugegeben. Man löst in ca. 1 mL CDCl₃, und entnimmt 0,5-0,75 mL für eine NMR-Messung. Die restliche Lösung wird zur präparativen Dünnschichtchromatographie verwendet. Mit dem isolierten Produkt aus dieser Dünnschichtchromatographie wird der Enantiomerenüberschuß mittels chiraler GC- oder chiraler HPLC-Messung bestimmt.

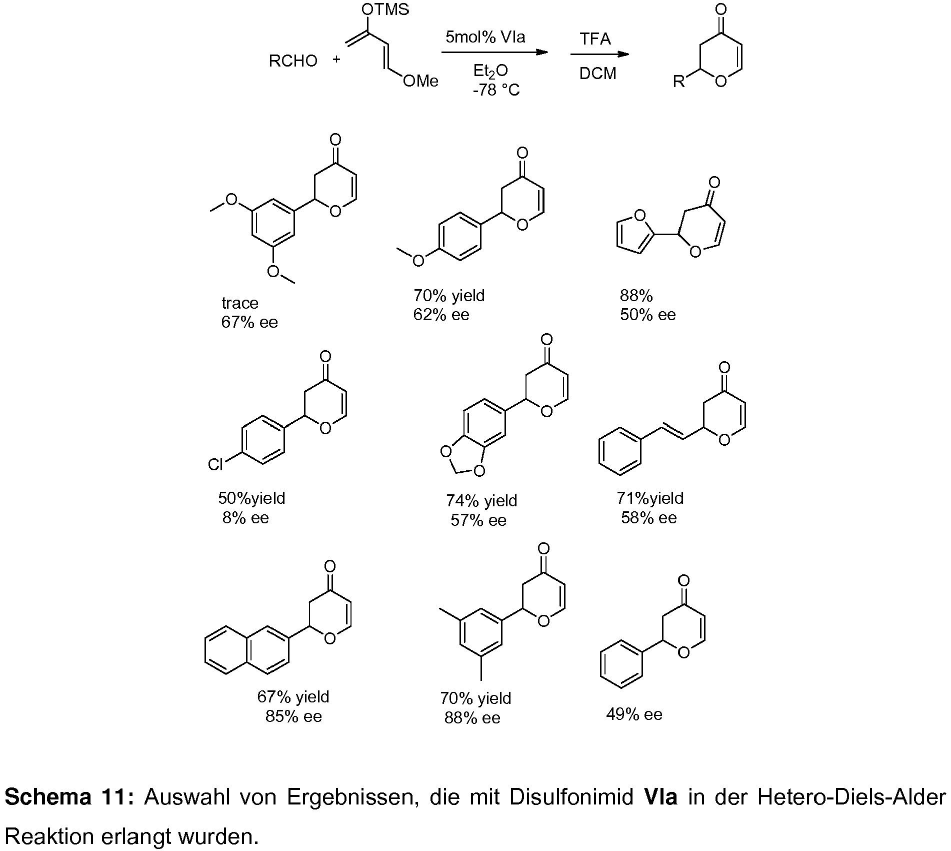

### Allgemeine Synthesevorschrift zur TMSCN Addition an Aldehyde

Disulfonimid **VIa** (2.05 mg, 5 mol%) und der entsprechende Aldehyd (0.05 mmol, 1 eq) werden in 0.25 ml Et₂O bei -25 °C vorgelegt. Man gibt TMSCN (0.15 mmol, 3 eq) hinzu und rührt bei -78 °C. Danach quencht man mit 10%iger TFA Lösung in Dichlormethan bei -25 °C. Das Lösungsmittel wird unter vermindertem Druck entfernt und 0.05 mmol Ph₃PH als interner Standard für eine NMR-Messung zur Ausbeutebestimmung hinzugegeben. Man löst in ca. 1 mL CDCl₃, und entnimmt 0,5-0,75 mL für eine NMR-Messung. Die restliche Lösung wird zur präparativen Dünnschichtchromatographie verwendet. Mit dem isolierten Produkt aus dieser Dünnschichtchromatographie wird der Enantiomerenüberschuß mittels chiraler GC- oder chiraler HPLC-Messung bestimmt.

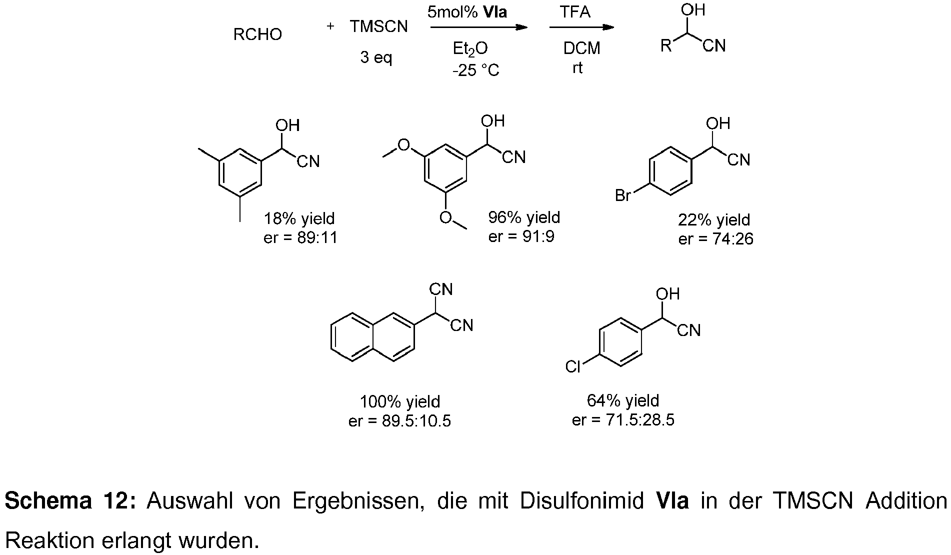

### Allgemeines Verfahren zur Darstellung der Disulfonimide

### a) Darstellung der O-Arylthiocarbamate XIII.

Das entsprechende Diol (1 eq) wird in DMF (0.2 M) bei 0°C oder Raumtemperatur vorgelegt. Man gibt 60%iges NaH in Öl (4 eq) zügig, aber portionsweise zu. Nach 5 min rühren bei Raumtemperatur wird *N,N*-Dimethylthiocarbamoylchlorid in DMF (6 mmol/ml) via Spritze zugegeben. Anschließend wird für 12-18h auf 85°C erhitzt. Nach Abkühlen versetzt man die Lösung mit 2%iger KOH. Der entstehende Niederschlag wird abgesaugt, mit 2%iger KOH nachgewaschen, in Dichlormethan gelöst und mit Wasser gewaschen. Die organische Phase wird über Na₂SO₄ getrocknet und eingeengt. Säulenchromatographie (SiO₄, Ethylacetat/Hexan) liefert das gewünschte *O*-Arylthiocarbamat.

### b) Darstellung der S-Arylthiocarbamate XV

Das *O*-Arylthiocarbamat wird unter Argon für 80-90 min auf 250°C erhitzt. Reinigung durch Säulenchromatographie (SiO₄, Ethylacetat/Hexan) liefert das gewünschte *S-*Arylthiocarbamat.

### c) Darstellung der Disulfonsäuren XVI

Essigsäure oder Ameisensäure (10faches Volumen von 30%iger H₂O₂) wird bei 0°C oder Raumtemperatur vorgelegt und 30%ige H₂O₂ (30 eq) tropfenweise zugegeben. Nach 1h rühren bei RT wird das S-Arylthiocarbamat, gelöst in Essigsäure, THF oder Dichlormethan (5faches Volumen von 30%iger H₂O₂), zugegeben. Nach rühren bei Raumtemperatur für 1-72h wird die Lösung eingeengt bzw. man filtriert die Lösung über Kieselgel und eingt danach ein. Säulenchromatographie (SiO₄, Dichlormethan/Methanol) liefert das Salz der gewünschten Säure. Dieses wird durch waschen mit 2-6M HCl oder mittels lonenaustauschchromatographie in die gewünschte Säure umgewandelt.

### d) Darstellung der Disulfonsäurechloride XIX

Die entsprechende Disulfonsäure wird in Thionylchlorid unter Argon vorgelegt. Nach Zugabe einer katalytischen Menge DMF wird für 1-4 h zum Rückfluß erhitzt. Man entfernt das Thionylchlorid unter Vakuum und reinigt durch digerieren mit Diethylether oder durch Säulenchromatographie (SiO₄, Hexan/Ethylacetat).

### d1) Darstellung der Disulfonsäurechloride XIX

Das entsprechende *S*-Arylthiocarbamat wird in einer Mischung von 2M HCl-Acetonitril (1:5) bei 0 °C suspendiert. Man gibt darauf *N*-Chlorsuccinimid (4-30 eq) portionsweise zu. Nach rühren für 10-240min bei knapp unter 20 °C wird die Suspension mit Diethylether extrahiert, mit ges. NaCl-Lösung gewaschen und eingeengt. Reinigung erfolgt mittels Säulenchromatographie (SiO₄, Ethylacetat/Pentan).

### e) Darstellung der Disulfonimide IV

Das entsprechende Disulfonsäurechlorid wird in THF gelöst. Man gibt Ammoniak (5-30eq) in Methanol portionsweise bzw. via Spritzenpumpe bei -10°C bis zu 60°C zu und rührt für 6-120h bei dieser Temperatur. Nach entfernen des Lösungsmittels erhält man das Salz des Disulfonimides durch Reinigung mittels Säulenchromatographie (SiO₄, Hexan/Ethylacetat). Dieses wird durch waschen mit 2-6M HCl oder mittels lonenaustauschchromatographie in das gewünschte Disulfonimid umgewandelt.

### e1) Darstellung der Disulfonimide IV über die Diboronate XXII oder die Dihalogenide XX.

Das entsprechende Disulfonimid **IVa** (R¹, R¹² = H bzw. R¹', R^{12'} = H) wird bei -78°C bis 0°C mit n-Buli, sec-BuLi oder tert-BuLi deprotoniert. Durch Zugabe einer Halogenidquelle oder einer Borquelle bei -78°C bis 0°C erhält man die Dihalogenide **XX** oder Diboronate **XXII.** Nach rühren bei Raumtemperatur für 1-120h wird die Lösung mit Ammoniumchlorid gequenscht. Man extrahiert die wässrige Phase mit einem geeigneten organischen Lösungsmittel. Die organische Phase wird über Na₂SO₄ getrocknet und eingeengt. Säulenchromatographie (SiO₄, Dichlormethan/Methanol) liefert das Salz des gewünschten Dihalogenides **XX** oder Diboronates **XXII.** Dieses Salz wird durch waschen mit 2-6M HCl oder mittels lonenaustauschchromatographie in die gewünschte Säure umgewandelt.

Das Diiodid wird mit der entsprechenden Boronsäure (2-20eq) und 1-40 mol% Pd(PPh₃)₄ unter Argon in einem entgastem Gemisch aus 2M Na₂CO_{3aq}. (2-30 eq) und DME für 1-72h auf 50-150 °C erhitzt. Nach Abkühlen des Gemisches verdünnt man mit Wasser. Man extrahiert mit Ethylacetat, trocknet die organischen Phasen über Na₂SO₄ und engt ein. Säulenchromatographie (SiO₄, Dichlormethan/Methanol) liefert das Salz der gewünschten Disulfonimide **IV.** Dieses Salz wird durch waschen mit 2-6M HCl oder mittels lonenaustauschchromatographie in die gewünschte Säure umgewandelt.

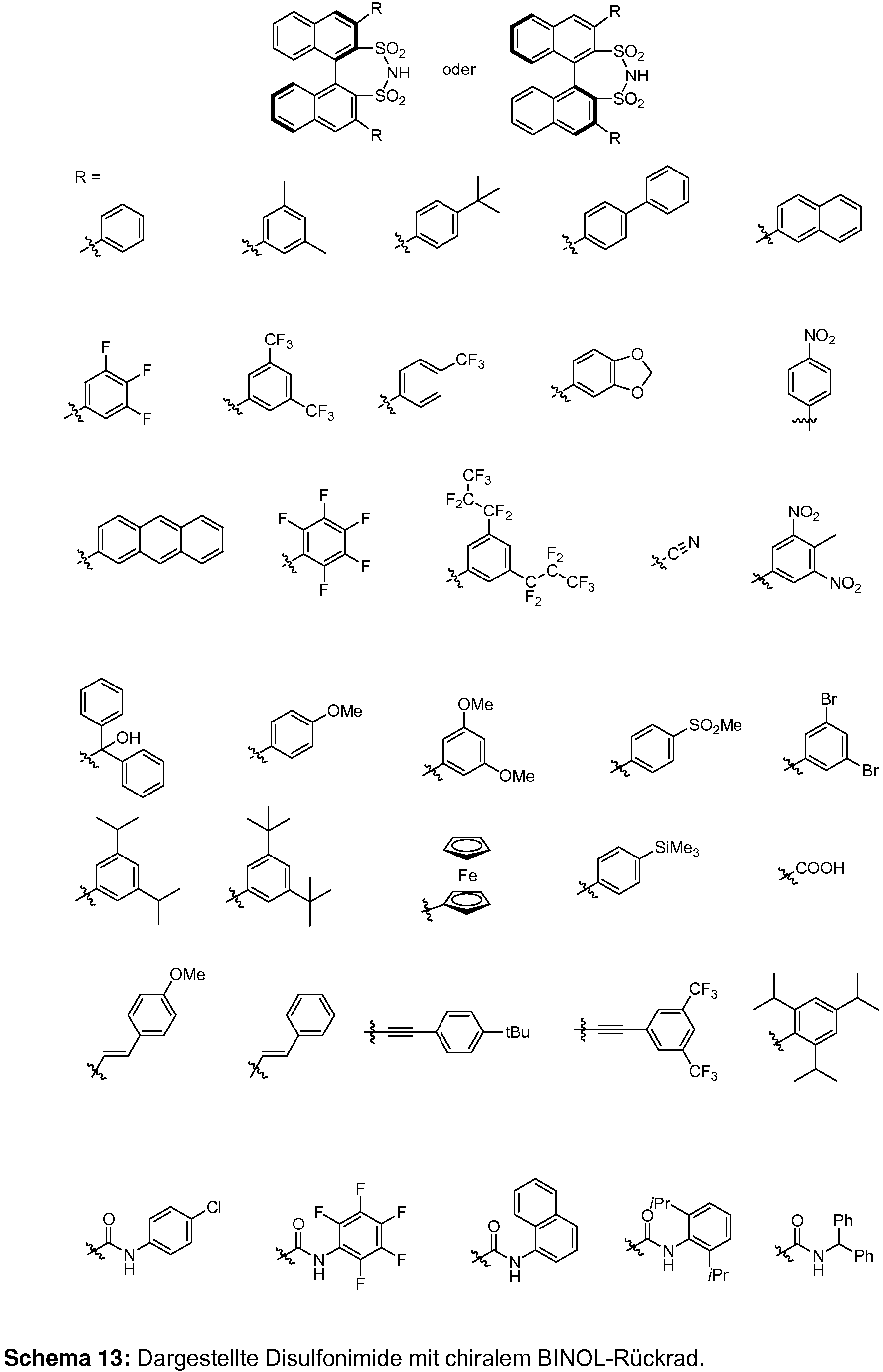

## Patentansprüche

1. Chirale Disulfonimide mit der allgemeinen Formel IV 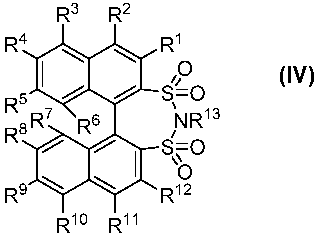 in der
R¹, R², R³, R⁴, R⁵, R⁶, R⁷, R⁸, R⁹, R¹⁰, R¹¹ und R¹² gleich oder verschieden sein können und unabhängig voneinander H, OH, F, Cl, Br, I, CN, NO₂, NO, SO₂, SO₃H, NH₂, PH₃, COOH, SO₃X, COOY, worin X und Y für Na oder K steht, eine C₁-C₂₀-Alkyl-, C₂-C₂₀-Alkenyl- oder C₂-C₂₀-Alkinyl-, Aryl-, Aryl-(C₁-C₆)-alkyl, Hetero(C₁-C_{y6})-alkyl-, Heteroaryl-Gruppe, die ggf. substituiert sein können, bedeuten,
R¹³ für H, eine C₁-C₆-Alkyl-, C₂-C₆-Alkenyl- oder C₂-C₆-Alkinyl-, Aryl-, Aryl-(C₁-C₆)-alkyl, Hetero(C₁-C₆)-alkyl-, Heteroaryl- oder SiR¹⁴R¹⁵R¹⁶-Gruppe steht, wobei R¹⁴, R¹⁵, R¹⁶ für C₁-C₆-Alkyl-, C₂-C₆-Alkenyl- oder C₂-C₆-Alkinyl-, Aryl-, Aryl-(C₁-C₆)-alkyl, Hetero-(C₁-C₆)-alkyl-, Heteroaryl, die ggf. substituiert sein können, steht,
sowie deren organische Salze, Metallsalze und Metallkomplexe.

2. Chirale Disulfonimide nach Anspruch 1, **dadurch gekennzeichnet, dass** mindestens einer von R¹ oder R¹² nicht Wasserstoff ist und ausgewählt ist aus Phenyl-, 2,4,6-Triisopropyl-phenyl, Mesityl, 9-Phenanthryl, 9-Anthracenyl, Ferrocenyl, N-(perfluorophenyl)acetamid, N-(4-chlorophenyl)acetamid, N-(naphthalen-1-yl) acetamid, N-benzhydrylacetamid, N-(2,6-diisopropylphenyl)acetamid, 1-Anthracenyl, Corannulen, Porphyrin, 1-Naphthyl, 2-Naphthyl, 4-Biphenyl, 3,5-(Trifluoromethyl)-phenyl, 2,6-Dimethyl-phenyl, tert-Butyl, tris-Mesityl-silyl, tris-Phenyl-silyl, 4-Nitrophenyl und 2,6-Methyl-4-butyl-phenyl, Trifluormethyl, unverzweigte (lineare) und verzweigte (C₁-C₁₂-Perfluoralkyle, 3,4,5-Trifluorphenyl, 1,3-bis(perfluoropropan-2-yl)-phenyl, 1,3-bis(perfluorobutyl)-phenyl und/oder Pentafluorphenyl sowie Chlorid, Jodid, Fluorid, B(OH)₂, B(alkyl)₂, B(Oalkyl)₂, B(pinakol), BF₃X X=Na oder K, OTf, MgCl, MgBr, ZnCl.

3. Chirale Disulfonimide nach einem der vorangehenden Ansprüche, **dadurch gekennzeichnet, dass** mindestens einer der Reste R⁴ und R⁹ ausgewählt aus NO₂ oder I ist.

4. Chirale Disulfonimide nach einem der Ansprüche 1 bis 3, **dadurch gekennzeichnet, dass** R¹³ für H, F, Cl, Br, I oder SiR¹⁴R¹⁵R¹⁶ steht, wobei R¹⁴, R¹⁵, R¹⁶ für C₁-C₆-Alkyl-, C₂-C₆-Alkenyl- oder C₂-C₆-Alkinyl ist.

5. Chirale Disulfonimide nach Ansprüche 4, **dadurch gekennzeichnet, dass** R¹³ für SiR¹⁴R¹⁵R¹⁶, worin R¹⁴, R¹⁵ und R¹⁶ wie in Anspruch 2 definiert sind, oder J steht.

6. Chirale Disulfonsäureimide nach einem der Ansprüche 1 bis 5, **dadurch gekennzeichnet, dass** die Enantiomeren der Verbindungen VI und VII 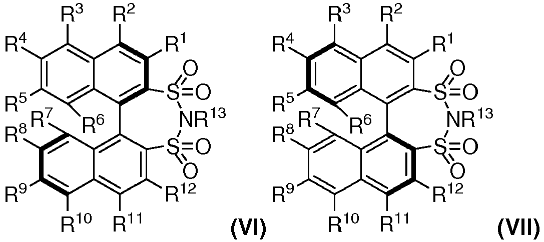 in denen die Reste R¹, R², R³, R⁴, R⁵, R⁶, R⁷, R⁸, R⁹, R¹⁰, R¹¹ und R¹² sowie R¹³ wie oben definiert sind, in einem Verhältnis von 98:2 bis 100:0 vorliegen.

7. Verfahren zur Herstellung von substituierten Disulfonimiden mit der allgemeinen Formel IV 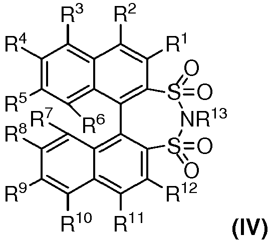 in der R¹, R², R³, R⁴, R⁵, R⁶, R⁷, R⁸, R⁹, R¹⁰ R¹¹, R¹² und R¹³ wie oben definiert sind, worin
(A) ein Binolderivat mit der allgemeinen Formel X 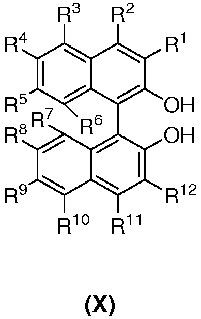 mit einem Thiocarbamoylchlorid der allgemeinen Formel XII 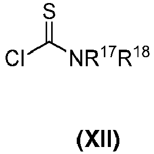 in der R¹⁷ und R¹⁸ gleich oder verschieden sein können und für eine C₁-C₆-Alkylgruppe stehen,
unter Bildung eines *O*-Arylthiocarbamates mit der allgemeinen Formel XIII 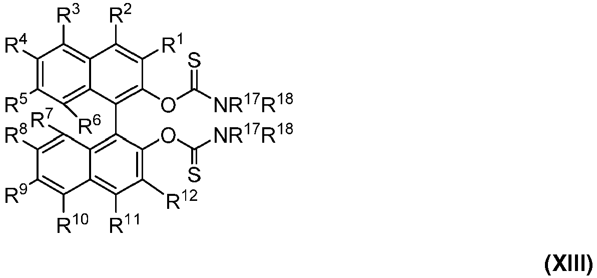 umgesetzt wird,
(B) das *O*-Arylthiocarbamat mit der Formel XIII in das entsprechende S-Arylthiocarbamat mit der Formel XV 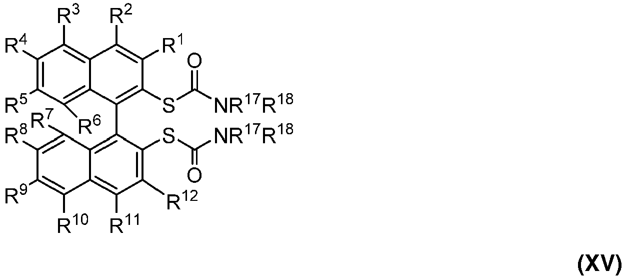 umgewandelt wird, und
(C) das S-Arylthiocarbamat in Gegenwart eines Oxidationsmittels in die Verbindung mit der Formel XVII umgewandelt wird 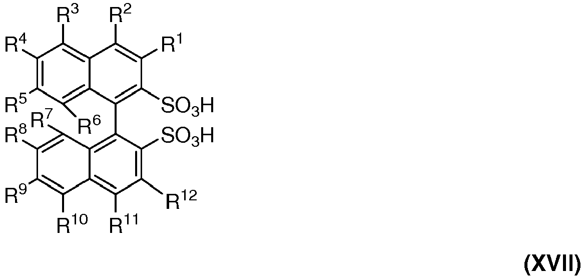
D) die in Schritt C erhaltenen Sulfonsäure in an sich bekannter Weise in das Säurehalogenid mit der Formel XIX 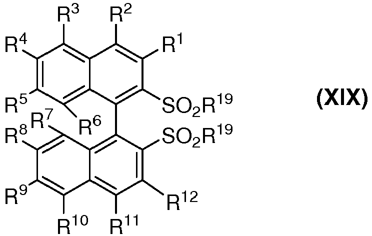 worin R¹⁹ = Cl, Br, I oder F bedeutet,
überführt wird und in einem nachfolgenden Verfahrensschritt
(E) mit Ammoniak oder einem primären Amin in das entsprechende Imid mit der Formel IV umgewandelt wird.

8. Verfahren zur Herstellung von substituierten Disulfonimiden mit der allgemeinen Formel IV, in welchem die Verfahrensschritte A bis B gemäß Anspruch 7 durchgeführt werden und das S-Arylthiocarbamat mit der Formel XV in einem Verfahrensschritt (D1) direkt in die Verbindung mit der Formel XIX umgewandelt wird.

9. Verfahren zur Herstellung von Verbindungen mit den allgemeinen Formel IV sowie deren organische Salze, Metallsalze und Metallkomplexe 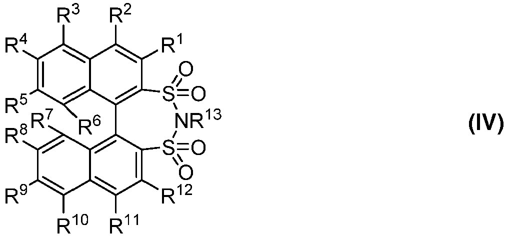 wobei R¹, R², R³, R⁴, R⁵, R⁶, R⁷, R⁸, R⁹, R¹⁰, R¹¹, R¹² und R¹³ wie in Anspruch 1 definiert sind,
in welchem
F) ein 3,3'-unsubstituiertes Disulfonimid mit der allgemeinen Formel IVa 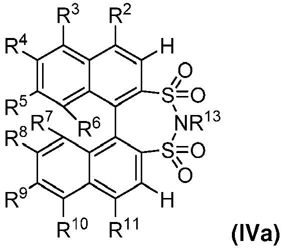 in ein 3,3'-Dihalogenid oder ein 3,3'-Ditriflat mit der allgemeinen Formel XX umgewandelt wird 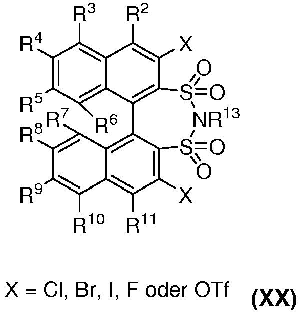 oder
G) das Disulfonimid IVa in ein 3,3'-Diboronat mit der allgemeinen Formel XXII umgewandelt wird 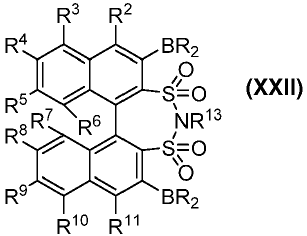 in dem die Reste BR² in XXII gleich oder verschieden sein können und für B(OH)₂, B(alkyl)₂, B(Oalkyl)₂, B(pinakol), BF₃X X₌Na, K bzw. R¹, R¹² = B(OH)₂, B(alkyl)₂, B(Oalkyl)₂, B(pinakol), BF₃X X=Na, K, stehen, und
H) in einem weiteren Verfahrensschritt die Verbindungen mit den allgemeinen Formeln XX oder XXII über Kupplungsreaktionen zu den Imiden mit der Formel IV überführt werden.

10. Verfahren zur Herstellung von Verbindungen mit der allgemeinen Formel IV sowie deren organische Salze, Metallsalze und Metallkomplexe 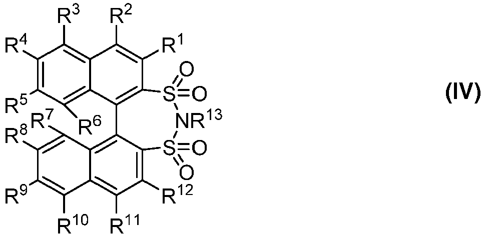 in dem R¹, R², R³, R⁴, R⁵, R⁶, R⁷, R⁸, R⁹, R¹⁰, R¹¹, R¹² und R¹³ wie in Anspruch 1 definiert sind, bei dem mit einer Verbindung der Formel (IVa) 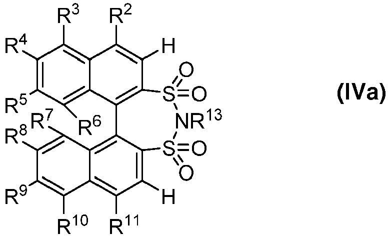 eine dirigierende ortho-Metallierung durchgeführt wird und die *in situ* gebildeten Metallspezies der Verbindung IV, in denen R¹, R¹² ₌ Li, Mg, Zn, Cu bedeuten, mit geeigneten Elektrophilen, wie CO₂, Perfluralkyliodiden oder Isocyanaten, Aldehyden oder Ketonen, umgesetzt werden.

11. Verwendung von chiralen Disulfonimiden mit der allgemeinen Formel IV 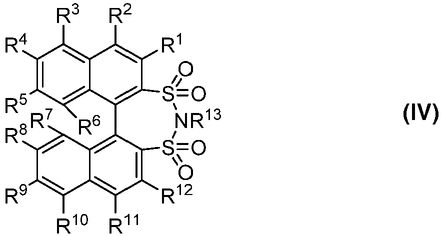 in der
R¹, R², R³, R⁴, R⁵, R⁶, R⁷, R⁸, R⁹, R¹⁰, R¹¹ und R¹² gleich oder verschieden sein können und unabhängig voneinander H, OH, F, Cl, Br, I, CN, NO₂, NO, SO₂, SO₃H, NH₂, PH₃, COOH, SO₃X, COOY, worin X und Y für Na oder K steht, eine C₁-C₂₀-Alkyl-, C₂-C₂₀-Alkenyl- oder C₂-C₂₀-Alkinyl-, Aryl-, Aryl-(C₁-C₆)-alkyl-, Hetero(C₁-C_{y6})-alkyl-, Heteroaryl-Gruppe, die ggf. substituiert sein können, bedeuten,
R¹³ für H, F, Cl, Br, I oder SiR¹⁴R¹⁵R¹⁶ steht, wobei R¹⁴, R¹⁵, R¹⁶ für C₁-C₆-Alkyl-, C₂-C₆-Alkenyl- oder C₂-C₆-Alkinyl-, Aryl-, Aryl-(C₁-C₆)-alkyl, Hetero-(C₁-C₆)-alkyl-, Heteroaryl, die ggf. substituiert sein können, steht, sowie Salze oder Metallkomplexe davon;
als chirale Brønsted-Säure-Katalysatoren oder chirale Lewis-Säure-Katalysatoren.

12. Verwendung nach Anspruch 11, **dadurch gekennzeichnet, dass** R¹³ für SiR¹⁴R¹⁵R¹⁶, worin R¹⁴, R¹⁵ und R¹⁶ wie in Anspruch 1 definiert sind, oder I steht.

13. Verwendung von Verbindungen mit den Formeln IV als NMR-Shift Reagenzien und als Reagenzien zur Racematspaltung.

14. Verwendung der Verbindungen mit den Formeln IV als chirale Brønsted-Säure-Katalysatoren bzw. chirale Lewis-Säure-Katalysatoren zur Aktivierung von Ketonen, Aldehyden und Alkenen, insbesondere als Katalysatoren in Aldol-Reaktionen, vinylogen Aldol-Reaktionen, Mukaiyama-Aldol-Reaktionen, vinylogen Mukaiyama-Aldol-Reaktionen Mukaiyama-Michael-Reaktionen, Michael-Additionen, Mannich-Reaktionen, TMSCN Additionen an Aldehyden und Ketonen, Veresterungen, Veretherungen, Pinakol-Umlagerungen, Acetalisierungen und verwandte Reaktionen, Cycloadditionen, Hydroaminierungen, Hydroalkoxilierung, Hydratisierungen, Olefinaktivierungen im Allgemeinen, Friedel-Crafts-Reaktionen, Epoxidöffnungen, Ritter-Reaktionen, nucleophilen Substitutionen von Alkoholen, asymmetrische Ringöffnungen, asymmetrische Reduktionen, transfer Hydrierungen, Alkin-Additionen, Allylierungen, Epoxidierungen, Olefin-Metathese, Isomerisierungen, Iminiumkatalyse und Enaminkatalyse.

## Claims

1. Chiral disulfonimides having the general formula 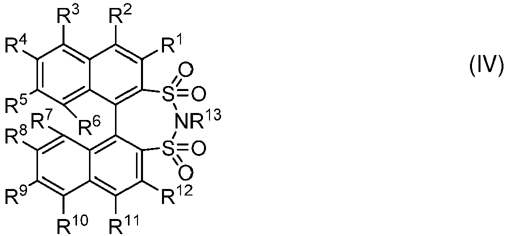 where
R¹, R², R³, R⁴, R⁵, R⁶, R⁷, R⁸, R⁹, R¹⁰, R¹¹ and R¹² can be identical or different and are each, independently of one another, H, OH, F, Cl, Br, I, CN, NO₂, NO, SO₂, SO₃H, NH₂, PH₃, COOH, SO₃X, COOY, where X and Y are each Na or K, a C₁-C₂₀-alkyl, C₂-C₂₀-alkenyl or C₂-C₂₀-alkynyl, aryl, aryl-(C₁-C₆)-alkyl, hetero(C₁-C_{y6})-alkyl, heteroaryl group, which may optionally be substituted,
R¹³ stands for H, an C₁-C₆-alkyl, C₂-C₆-alkenyl or C₂-C₆-alkynyl, aryl, aryl-(C₁-C₆)-alkyl, hetero(C₁-C₆)-alkyl, heteroaryl- or SiR¹⁴R¹⁵R¹⁶-group, where R¹⁴, R¹⁵, R¹⁶ are each C₁-C₆-alkyl, C₂-C₆-alkenyl or C₂-C₆-alkynyl, aryl, aryl-(C₁-C₆)-alkyl, hetero(C₁-C₆)-alkyl, heteroaryl, which may optionally be substituted,
or organic salts, metal salts or metal complexes thereof.

2. The chiral disulfonimide as claimed in claim 1, **characterized in that** at least one of R¹ or R¹² is not hydrogen and is selected from among phenyl-, 2,4,6-triisopropylphenyl, mesityl, 9-phenanthryl, 9-anthracenyl, ferrocenyl, N-(perfluorophenyl)acetamide, N-(4-chlorophenyl)acetamide, N-(naphthalen-1-yl)acetamide, N-benzhydrylacetamide, N-(2,6-diisopropylphenyl)acetamide, 1-anthracenyl, corannulene, porphyrin, 1-naphthyl, 2-naphthyl, 4-biphenyl, 3,5-(trifluoromethyl)phenyl, 2,6-dimethylphenyl, tert-butyl, tris-mesitylsilyl, tris-phenylsilyl, 4-nitrophenyl and 2,6-methyl-4-butylphenyl, trifluoromethyl, unbranched (linear) and branched (C₁-C₁₂)-perfluoroalkyls, 3,4,5-trifluorophenyl, 1,3-bis(perfluoropropan-2-yl)phenyl, 1,3-bis(perfluorobutyl)phenyl and/or pentafluorophenyl and also chloride, iodide, fluoride, B(OH)₂, B(alkyl)₂, B(Oalkyl)₂, B(pinacol), BF₃X where X=Na or K, OTf, MgCl, MgBr, ZnCl.

3. The chiral disulfonimide as claimed in any of the preceding claims, **characterized in that** at least one of the radicals R⁴ and R⁹ is selected from among NO₂ and I.

4. The chiral disulfonimide as claimed in any of claims 1 to 3, **characterized in that** R¹³ is H, F, Cl, Br, I or SiR¹⁴R¹⁵R¹⁶, where R¹⁴, R¹⁵, R¹⁶ are each C₁-C₆-alkyl, C₂-C₆-alkenyl or C₂-C₆-alkynyl.

5. The chiral disulfonimide as claimed in claim 4, **characterized in that** R¹³ is SiR¹⁴R¹⁵R¹⁶, where R¹⁴, R¹⁵ and R¹⁶ are as defined in claim 2, or I.

6. The chiral disulfonimide as claimed in any of claims 1 to 5, **characterized in that** the enantiomers of the compound VI or VII 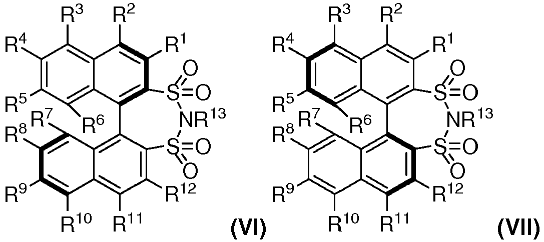 where the radicals R¹, R², R³, R⁴, R⁵, R⁶, R⁷, R⁸, R⁹, R¹⁰, R¹¹ and R¹² and also R¹³ are as defined above, are present in a ratio of from 98:2 to 100:0.

7. A process for preparing substituted disulfonimides having the general formula IV 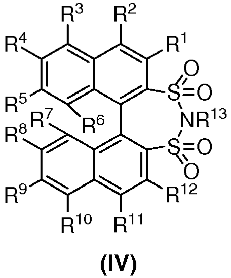 where
R¹, R², R³, R⁴, R⁵, R⁶, R⁷, R⁸, R⁹, R¹⁰, R¹¹, R¹² and R¹³ are as defined above,
wherein
(A) a Binol derivative having the general formula X 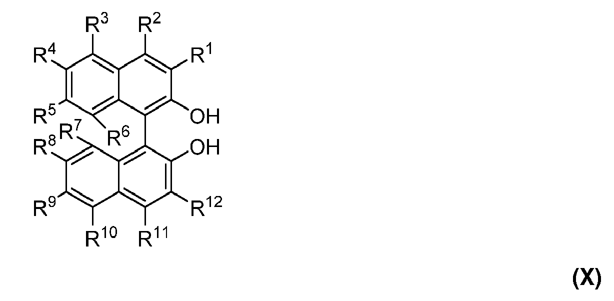 is reacted with a thiocarbamoyl chloride of the general formula XII 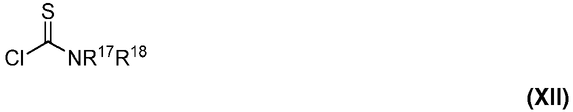 where R¹⁷ and R¹⁸ can be identical or different and are each a C₁-C₆-alkyl group, to form an *O*-arylthiocarbamate having the general formula XIII, 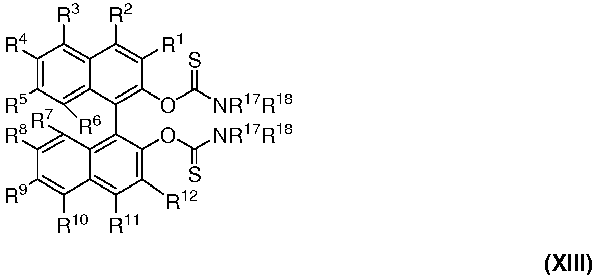
(B) the *O*-arylthiocarbamate having the formula XIII is converted into the corresponding S-arylthiocarbamate having the formula XV, 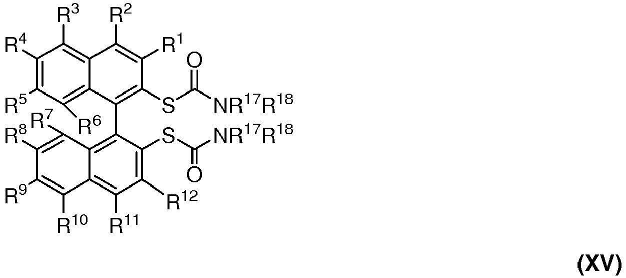 and
(C) the S-arylthiocarbamate is converted in the presence of an oxidant into the compound having the formula XVII, 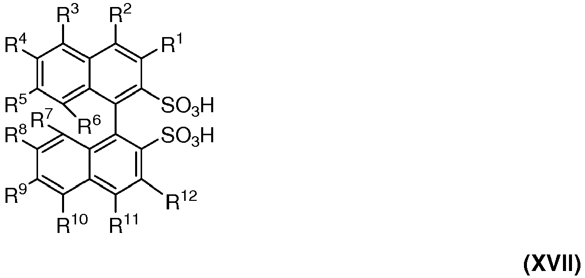
D) the sulfonic acid obtained in step C is converted in a manner known per se into the acid halides having the formula XIX, 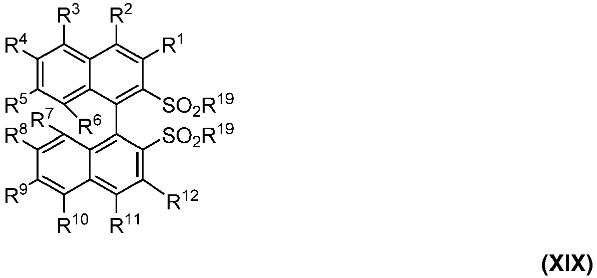 where R¹⁹ = Cl, Br, I or F,
and in a subsequent process step,
(E) is converted by means of ammonia or a primary amine into the corresponding imide having the formula IV.

8. A process for preparing substituted disulfonimides having the general formula IV, wherein the process steps A to B as claimed in claim 7 are carried out and the S-arylthiocarbamate having the formula XV is converted directly in one step (D1) into the compound having the formula XIX.

9. A process for preparing compounds having the general formulae IV and their organic salts, metal salts and metal complexes 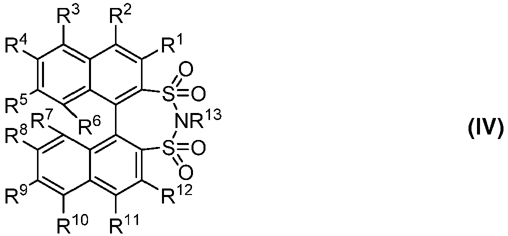 R¹, R², R³, R⁴, R⁵, R⁶, R⁷, R⁸, R⁹, R¹⁰, R¹¹, R¹² and R¹³ are as defined in claim 1,
wherein
F) a 3,3'-unsubstituted disulfonimide having the general formula IVa 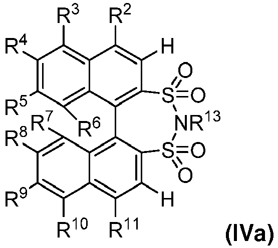 is converted into a 3,3'-dihalide or a 3,3'-ditriflate having the general formula XX 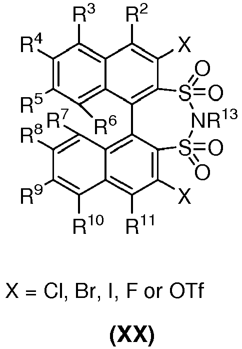 or
G) the disulfonimide IVa is converted into a 3,3'-diboronate having the general formula XXII 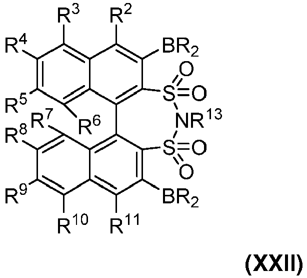 where the radicals BR² in XXII can be identical or different and are each B(OH)₂, B(alkyl)₂, B(Oalkyl)₂, B(pinacol), BF₃X, where X=Na, K, or R¹, R¹² are B(OH)₂, B(alkyl)₂, B(Oalkyl)₂, B(pinacol), BF₃X, where X=Na, K, resp., and
H) in a further process step, the compounds having the general formulae XX or XXII are converted by means of coupling reactions into the imides having the formula IV.

10. A process for preparing compounds having the general formula IV and their organic salts, metal salts and metal complexes: 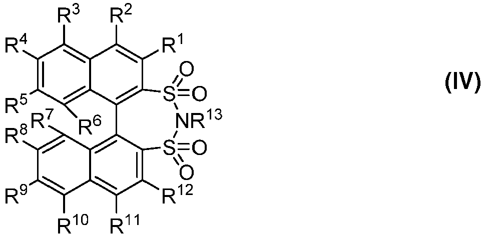 Wherein R¹, R², R³, R⁴, R⁵, R⁶, R⁷, R⁸, R⁹, R¹⁰, R¹¹, R¹² and R¹³ are as defined in claim 1,
wherein 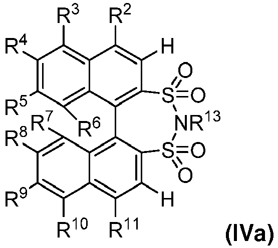 a directing ortho-metallization is carried out with a compound of the formula (IVa) and the metal species of the compounds IV formed *in situ,* in which R¹, R¹² = Li, Mg, Zn, Cu are reacted with suitable electrophiles such as CO₂, perfluoroalkyl iodides or isocyanates, aldehydes or ketones.

11. The use of chiral disulfonimides having the general formula IV 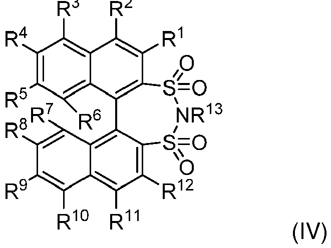 Where
R¹, R², R³, R⁴, R⁵, R⁶, R⁷, R⁸, R⁹, R¹⁰, R¹¹, and R¹² can be identical or different and are each, independently of one another, H, OH, F, Cl, Br, I, CN, NO₂, NO, SO₂, SO₃H, NH₂, PH₃, COOH, SO₃X, COOY, where X and Y are each Na or K, a C₁-C₂₀-alkyl, C₂-C₂₀-alkenyl or C₂-C₂₀-alkynyl, aryl, aryl-(C₁-C₆)-alkyl, hetero(C₁-C_{y6})-alkyl, heteroaryl group, which may optionally be substituted,
R¹³ is H, F, Cl, Br, I or SiR¹⁴R¹⁵R¹⁶, where R¹⁴, R¹⁵, R¹⁶ are each C₁-C₆-alkyl, C₂-C₆-alkenyl or C₂-C₆-alkynyl, aryl, aryl-(C₁-C₆)-alkyl, hetero(C₁-C₆)-alkyl, heteroaryl, which may optionally be substituted, and salts or metal complexes thereof,
as chiral Brønsted acid catalysts or chiral Lewis acid catalysts.

12. The use as claimed in claim 11, **characterized in that** R¹³ is SiR¹⁴R¹⁵R¹⁶, where R¹⁴, R¹⁵ and R¹⁶ are as defined in claim 1, or I.

13. The use of compounds having the formula (IV) as NMR shift reagents and as reagents for the resolution of racemates.

14. The use of the compounds having the formula (IV) as chiral Brønsted acid catalysts or chiral Lewis acid catalysts for the activation of ketones, aldehydes and alkenes, in particular as catalysts in aldol reactions, vinylic aldol reactions, Mukaiyama aldol reactions, vinylic Mukaiyama aldol reactions, Mukaiyama-Michael reactions, Michael additions, Mannich reactions, TMSCN additions onto aldehydes and ketones, esterifications, etherifications, pinacol rearrangements, as acetalizations and related reactions, cycloadditions, hydroaminations, hydroalkoxylation, hydrations, olefin activations in general, Friedel-Crafts reactions, epoxide openings, Ritter reactions, nucleophilic substitutions of alcohols, asymmetric ring openings, asymmetric reductions, transfer hydrogenations, alkyne additions, allylations, epoxidations, olefin metathesis, isomerizations, iminium catalysis and enamine catalysis.

## Revendications

1. Disulfonimides chiraux de la formule générale V 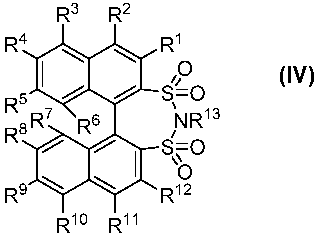 dans laquelle
R¹, R², R³, R⁴, R⁵, R⁶, R⁷, R⁸, R⁹, R¹⁰, R¹¹ et R¹² peuvent être identiques ou différents et signifient, indépendamment l'un de l'autre H, OH, F, Cl, Br, I, CN, NO₂, NO, SO₂, SO₃H, NH₂, PH₃, COOH, SO₃X, COOY, X et Y correspondant au Na ou au K, à un groupe alkyle en C₁ à C₂₀, un groupe alkényle en C₂ à C₂₀ ou un groupe alkinyle en C₂ à C₂₀, un groupe aryle, un groupe arylalkyle en (C₁ à C₆), un groupe heteroalkyle en C₁ à C_{y6}), un groupe heteroaryle, qui peuvent être substitués le cas échéant,
R¹³ est H, un groupe alkyle en C₁ à C₆, un groupe alkényle en C₂ à C₆ ou un groupe alkinyle en C₂ à C₆, un groupe aryle, un groupe arylalkyle en (C₁ à C₆), un groupe heteroalkyle en (C₁ à C₆), un groupe heteroaryle où SiR¹⁴R¹⁵R¹⁶, R¹⁴, R¹⁵, R¹⁶ est C₁ un groupe alkyle en C₁ à C₆, un groupe alkényle en C₂ à C₆ ou un groupe alkinyle en C₂ à C₆, un groupe aryle , un groupe arylalkyle en (C₁ à C₆), un groupe heteroalkyle en (C₁ à C₆), un groupe heteroaryle qui peuvent être substitués le cas échéant,
ainsi que leurs sels organiques, sels métalliques ou complexes métalliques.

2. Disulfonimides chiraux selon la revendication 1, **caractérisés en ce qu'**au moins l'un de R¹ ou R¹² n'est pas de l'hydrogène et est choisi parmi un phénylphényle, un 2,4,6 triisopropylphényle, un mésityle, un 9-phénanthryle, un 9-anthracényle, un ferrocényle, un n-(perfluorophényl)acétamide, un n-(4 à chlorophényl)acétamide, un n-(naphtalène-1-yl)acétamide, un n-benzhydrylacétamide, un n-(2,6-diisopropylphényl)acétamide, un 1-anthracényle, un corannulène, une porphyrine, un 1-naphthyle, un 2-naphthyle, un 4-biphényle, un 3,5-(trifluorométhyl)-phényle, un 2,6-diméthylphényle, un tert-butyle, un tris-mésitylsilyle, un tris-phénylsilyle, un 4-nitrophényle et un 2,6-méthyl-4-butyl-phényle, un trifluorométhyle, un phényle non ramifié (linéaire) et ramifié d'un phényle de (perfluoroalkyle en C₁ à C₁₂, un 3,4,5-trifluorphényle, un 1,3- bis(perfluoropropan-2-yle), un 1,3 bisphényle (perfluorobutyl) et/ou un pentafluorophényle ainsi qu'un chlorure, iodure, fluorure, B(OH)₂, B(alkyl)₂, B(Oalkyl)₂, B(pinacole), BF₃X X=Na ou K, OTf, MgCl, MgBr, ZnCl.

3. Disulfonimides chiraux selon l'une quelconque des revendications précédentes, **caractérisés en ce que** l'au moins un des radicaux R⁴ et R⁹ est choisi parmi le NO₂ ou I'I.

4. Disulfonimides chiraux selon l'une quelconque des revendications 1 à 3, **caractérisés en ce que** R¹³ signifie H, F, Cl, Br, I ou SiR¹⁴R¹⁵R¹⁶, alors que R¹⁴, R¹⁵, R¹⁶ est un alkyle en C₁ à C₆, un alkényle en C₂ à C₆ ou un alkinyle en C₂ à C₆.

5. Disulfonimides chiraux selon la revendication 4, **caractérisé en ce que** R¹³ signifie SiR¹⁴R¹⁵R¹⁶, alors que R¹⁴, R¹⁵ R¹⁶ sont définis comme dans la revendication 2 ou signifient J.

6. Imides d'acide sulfonique chiraux selon l'une quelconque des revendications 1 à 5, **caractérisés en ce que** les énantiomères des composés VI et VII 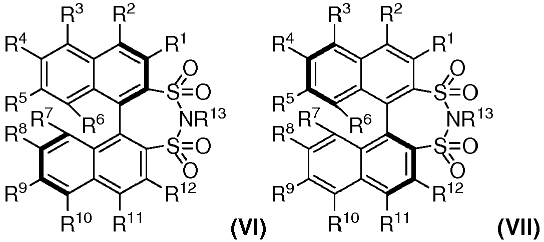 dans lesquels les radicaux R¹, R², R³, R⁴, R⁵, R⁶, R⁷, R⁸, R⁹, R¹⁰, R¹¹ et R¹², ainsi que R¹³ sont définis comme ci-dessus sont présents selon un rapport de 98:2 à 100:0.

7. Procédé destiné à préparer des disulfonimides substitués selon la formule générale IV 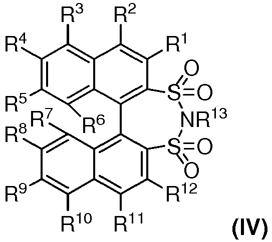 dans laquelle R¹, R², R³, R⁴, R⁵, R⁶, R⁷, R⁸, R⁹, R¹⁰, R¹¹, R¹² et R¹³ sont définis comme ci-dessus,
dans laquelle
(A) on transforme un dérivé du binol de la formule générale X 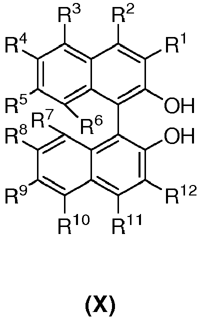 avec un chlorure de thiocarbamoyle de la formule générale XII 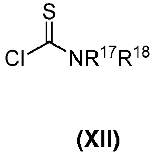 dans laquelle R¹⁷ et R¹⁸ peuvent être identiques ou différents et correspondent à un groupe alkyle en C₁ à C₆,
en formant un *O*-arylthiocarbamate de la formule générale XIII 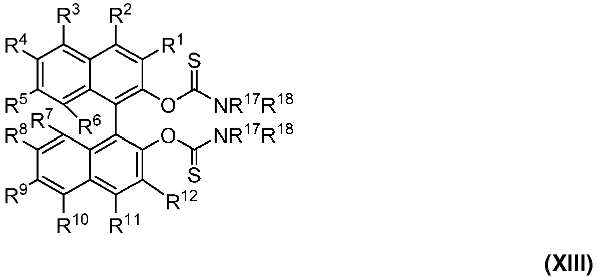
(B) on convertit l'*O*-arylthiocarbamate de la formule XIII dans le S-arylthiocarbamate de la formule XV 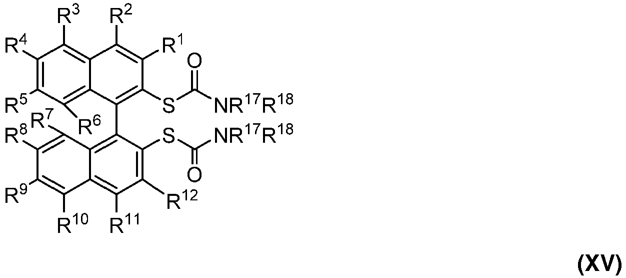 et
(C) on convertit le S-arylthiocarbamate en présence d'un agent oxydant dans le composé de la formule XVII 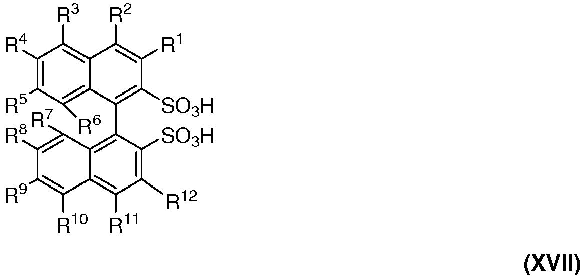
D) on réduit l'acide sulfonique obtenu dans l'étape C de manière connue en soi à l'halogénure d'acide de la formule XIX 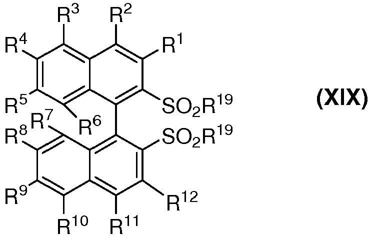 dans laquelle R¹⁹ ₌ signifie Cl, Br, I ou F,
et dans une étape de procédé suivante
(E) on le convertit avec de l'ammoniac ou un amine primaire dans l'imide correspondant de la formule IV.

8. Procédé destiné à préparer des disulfonimides de la formule générale IV, lors duquel on réalise les étapes de procédé A à B selon la revendication 7 et on convertit le S-arylthiocarbamate de la formule XV lors d'une étape de procédé (D1) directement dans le composé de la formule XIX.

9. Procédé destiné à préparer des composés de la formule générale IV, ainsi que leurs sels organiques, sels métalliques et complexes métalliques 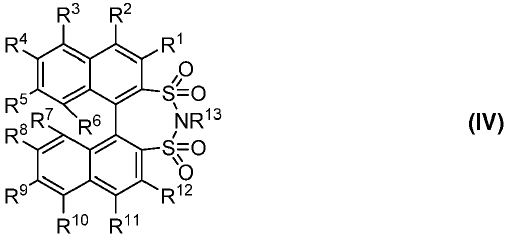 dans laquelle R¹, R², R³, R⁴, R⁵, R⁶, R⁷, R⁸, R⁹, R¹⁰, R¹¹, R¹² et R¹³ sont définis comme dans la revendication 1,
dans laquelle
F) on convertit un 3,3-disulfonimide non substitué de la formule générale IVa 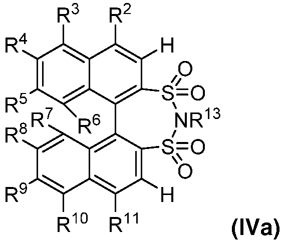 en un 3,3'-dihalogénure ou en un 3,3'-ditriflate de la formule générale XX 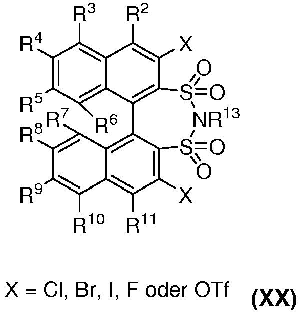 ou
G) on convertit le disulfonimide IVa en un 3,3'-diboronate de la formule générale 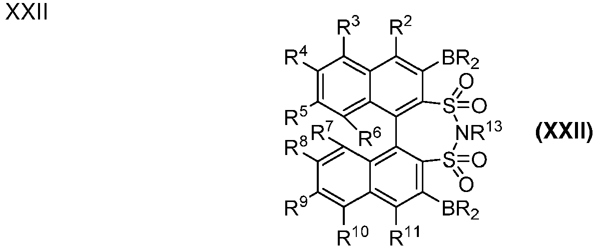 dans laquelle les radicaux BR² dans XXII peuvent être identiques ou différents et signifient B(OH)₂, B(alkyle)₂, B(Oalkyle)₂, B(pinacole), BF₃X X₌Na, K, ou R¹, R¹² ₌ B(OH)₂, B(alkyle)₂, B(Oalkyle)₂, B(pinacole), BF₃X X=Na, K, et
H) dans une étape de procédé supplémentaire, on réduit les composés de la formule générale XX ou XXII via des réactions de couplage aux imides de la formule IV .

10. Procédé destiné à préparer des composés de la formule générale IV, ainsi que leurs sels organiques, sels métalliques et complexes métalliques 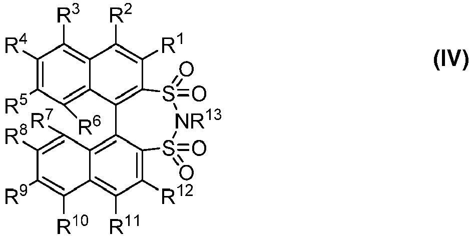 dans laquelle R¹, R², R³, R⁴, R⁵, R⁶, R⁷, R⁸, R⁹, R¹⁰, R¹¹, R¹² et R¹³ sont définis comme dans la revendication 1, lors duquel avec un composé de la formule (IVa) 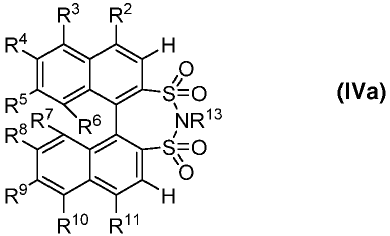 on procède à une orthométallisation et on transforme les espèces métalliques formées *in situ* du composé IV, dans laquelle R¹, R¹² = signifient Li, Mg, Zn, Cu avec des électrophiles adaptés comme le CO₂, des iodures de perfluoroalkyle ou des isocyanates, des aldéhydes ou des cétones.

11. Utilisation de disulfonimides de la formule générale IV 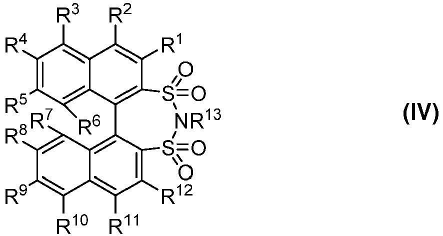 dans laquelle
R¹, R², R³, R⁴, R⁵, R⁶, R⁷, R⁸, R⁹, R¹⁰, R¹¹ et R¹² peuvent être identiques ou différents et indépendamment l'un de l'autre, signifient H, OH, F, Cl, Br, I, CN, NO₂, NO, SO₂, SO₃H, NH₂, PH₃, COOH, SO₃X, COOY, où X et Y correspondent à Na ou à K, à un groupe alkyle en C₁ à C₂₀, un groupe alkényle en C₂ à C₂₀ ou un groupe alkinyle en C₂ à C₂₀, un groupe aryle, un groupe arylalkyle en (C₁ à C₆), un groupe heteroalkyle en (C₁ à C_{y6}), un groupe heteroaryle, qui peuvent être substitués le cas échéant,
R¹³ est H, F, Cl, Br, I ou SiR¹⁴R¹⁵R¹⁶, sachant que R¹⁴, R¹⁵, R¹⁶ correspond à un groupe alkyle en C₁ à C₆, un groupe alkényle en C₂ à C₆ ou à un groupe alkinyle en C₂ à C₆, , à un groupe aryle , à une groupe arylalkyle en (C₁ à C₆), à un groupe heteroalkyle en (C₁ à C₆), à un groupe heteroaryle qui peuvent être substitués le cas échéant, ainsi que des sels ou des complexes métalliques de ces derniers;
en tant que catalyseurs acides de Brønsted chiraux ou que catalyseurs acides de Lewis chiraux.

12. Utilisation selon la revendication 11, **caractérisée en ce que** R¹³ correspond à SiR¹⁴R¹⁵R¹⁶, où R¹⁴, R¹⁵ et R¹⁶ sont définis comme dans la revendication 1 ou à I.

13. Utilisation de composés des formules IV en tant que réactifs RMN-Shift et en tant que réactifs pour une dissociation racémique.

14. Utilisation de composés des formules IV en tant que catalyseurs acides de Brønsted chiraux ou que catalyseurs acides de Lewis chiraux pour l'activation de cétones, d'aldéhydes, et d'alcènes, notamment en tant que catalyseurs dans des réactions aldoliques, des réactions aldoliques vinylogènes, des réactions aldoliques de Mukaiyama, des réactions aldoliques vinylogènes de Mukaiyama, des réactions de Mukaiyama-Michael, des additions de Michael, des réactions de Mannich, des additions du TMSCN sur des aldéhydes et des cétones, des esthérisations, des éthérifications, des transpositions de pinacole, des acétylisations et des réactions apparentées, des cycloadditions, des hydroaminations, hydroalcooxylations, hydratations, des activations d'oléfine en général, des réactions de Friedel- Crafts, des ouvertures de cycle époxyde, des réactions de Ritter, des substitutions nucléophiles, des substitutions d'alcools, des décyclisations asymétriques, des réductions asymétriques, des hydrogénations de transfert, des additions d'alcyne, des allylations, des époxydations, des métathèses d'oléfine, des isomérisations, des catalyses d'iminium et des catalyses d'énamine.
